(19) Europäisches Patentamt
European Patent Office
Office européen des brevets

(11) EP 2 966 182 B1

(12) EUROPEAN PATENT SPECIFICATION

(45) Date of publication and mention of the grant of the patent:
**30.08.2017 Bulletin 2017/35**

(51) Int Cl.:
***C12Q 1/68*** *(2006.01)*

(21) Application number: **15176248.1**

(22) Date of filing: **10.07.2015**

(54) **METHOD FOR OBTAINING INFORMATION ON UTERINE BODY CANCER AND MARKER AND DETERMINATION DEVICE FOR OBTAINING INFORMATION ON UTERINE BODY CANCER**

VERFAHREN ZUM ERHALT VON INFORMATIONEN ÜBER UTERINKÖRPERKREBS UND MARKER UND BESTIMMUNGSVORRICHTUNG ZUM ERHALT VON INFORMATIONEN ÜBER DEN UTERINKÖRPERKREBS

PROCÉDÉ D'OBTENTION D'INFORMATIONS SUR UN CANCER DU CORPS UTÉRIN ET UN MARQUEUR DE CANCER ET DISPOSITIF DE DÉTERMINATION PERMETTANT D'OBTENIR DES INFORMATIONS SUR UN CANCER DU CORPS UTÉRIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.07.2014 JP 2014142497**

(43) Date of publication of application:
**13.01.2016 Bulletin 2016/02**

(73) Proprietors:
- **SYSMEX CORPORATION**
  **Kobe-shi,**
  **Hyogo 651-0073 (JP)**
- **The University of Tokyo**
  **Bunkyo-ku,**
  **Tokyo 113-8654 (JP)**

(72) Inventors:
- **Sakai, Ayako**
  **Kobe-shi**
  **Hyogo 651-0073 (JP)**
- **Tai, Kaya**
  **Kobe-shi**
  **Hyogo 651-0073 (JP)**
- **Nagae, Genta**
  **Tokyo 113-8654 (JP)**
- **Aburatani, Hiroyuki**
  **Tokyo 113-8654 (JP)**

(74) Representative: **Hoffmann Eitle**
**Patent- und Rechtsanwälte PartmbB**
**Arabellastraße 30**
**81925 München (DE)**

(56) References cited:
**WO-A1-2014/046200 US-A1- 2011 223 616**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> SYSMEX CORPORATION
THE UNIVERSITY OF TOKYO

<120> METHOD FOR OBTAINING INFORMATION ON UTERINE BODY CANCER AND MARKER AND DETERMINATION DEVICE FOR OBTAINING INFORMATION ON UTERINE BODY CANCER

<130> 13-042JP

<150> JP 2014-142497
<151> 2014-07-10

<160> 12

<170> PatentIn version 3.5

<210> 1
<211> 4240
<212> DNA
<213> Homo sapiens

<400> 1

```
cctatgacat ggaggtaata tttatacgag taaccacaaa tggttgctgt gtagatcaaa        60
tatggtaatt taagaaaaaa aacttcaaac ccctatgaag atataagaaa aagtcacaga       120
atacagaaag ttgtaggaat tctaagaaac aaaagttata tttaatcctt ataaggaaaa       180
aaagaagaga gaaaactaaa tcaggcctat aaggaaccat tcaccctgta cctttagctg       240
agtcctgtgg aattcctgct ttatttcaaa ggtaacaaac ccttcttgtt tttaatcttt       300
tccttaaaca cctcttcaac tttctaaccc aactgaaact cggcttatct ctgaagacac       360
cacttacctt gacaccagtc ataatgacat atcatcctcc aaacatgcct taaatgttaa       420
gatttccata tttttgctca tatgttattt ctttacctgg aatttccttt ccccatttca       480
ttctctatcc aaattctaac tatccacaag gaactgcaag gccatcagca tcatgaaggc       540
taccatggtt cctcacaact gaatgaagcc tcttcttctt ttttttttaa agacaagtct       600
cactattgcc caggaggcag tgtagtgaca ccatcgtggc ccactgtagc ctcaatctcc       660
tgggctcaag ggatcctcat gcctcagtct cccaaatagc tgggactata ggtgtgtacc       720
accataccca gctaattttt tctgtaggga cgaggtctcc ctatgttgcc caggctggtg       780
tcaaacccct aagttcagtc ctgcctcagc cttccaaagt gctgggatta acaggagtaa       840
gccactgcgc cagattgaac ctcttcttta gaaccaccac agcacataac atgtatctgc       900
tacgcccctc acttccctct agttggtatt acacattttg cataaaagtc ttctatttct       960
aaataggctt caagtcaggt tcatctttgt atcagctggt gctcttaaaa tatttattgt      1020
gaacatggac ttaatgagct tgacgtgttg aaattatttc taatgtgttg ccatactcac      1080
atttttact ctgtttttcc acttctgcct tcagtctctt gtacttgtct gtcctgtaaa      1140
ccaggaccca ggttatgcct aaaacattaa aagcaacatg ttaatagaga aatgactgga      1200
```

```
atgatcacaa caaacaaagt ggttactgtt ggagaaggag gaattccaac ttttcgcttt     1260
gtatttaccc atagttaact tttttcaaag gctaatacaa ctgactcttc agccaaaaca     1320
acagtaacat gatgtgaagg tgtctccaca aatacccaag tgaaatctac ctgagaccaa     1380
agaaaactcg gcaatcgact ccatactccc ctcctgctcc tgttcacgag ttgcccagag     1440
attccctgaa gtggagtaga tgagcctctc aagcaagtaa ggctcgcgat ctttcccctg     1500
gtggcacagg ggaccccggg gcccttcctc ccggggactg atcaaacgtc tgagaaatac     1560
ccgctctcac cctctgcgag cagagccgtg cacacagaga taaaaacgat gaggagagtg     1620
tccgcgaaca tagtgctcat ctcgcacctt cgtctctgca ctctcacccg ccagggggaa     1680
agcgctctac agccaggaaa agtgaagcga aaacggcttc cgtagactcc gccaccaccg     1740
agtaacagac caactctgac agcccgaaga tcgcacttcc gcttccgggg catagcaccg     1800
gaaaggctcg tatcggagaa cggtgtggcc acgagagtct agaggaaacc aacccgcaac     1860
tcactggagc tgaagggctg gtagtcaggc cgaatctcgc gagaattgga actatttcag     1920
agccagagga ggatagtggc ggcggcgaac tttgcccggt agattttact gactggggtg     1980
ctggcgcgcg ccttttctc ggccagaatc cagttattgg gatagtcttg acttctacct      2040
cagcccaact tctgtggtcg taatattagt ttagcttggg tttgccgtga agtaagaaga     2100
ctgacttccg caaaggtact gaacgactgg gtgtaggtgc cagggcatgg acgtgaagct     2160
ggaaccgcga aatgagttag ttgtgggccg ctggcctcaa ggagcgaacg tcagcgcgaa     2220
gaggagaagc ttgtgcatac gctctgataa gggttttaat agaggtatta ccacagtgct     2280
gcgtgacacg gttgagagag tggtcagaag aagctaatga aaggaattga agcagtgcct     2340
taggggagta ggagtacttt tggagatagc aaaggacttt ctaggcaaag tggaagtcaa     2400
agcacggatg cctgaaaatt catagtgtga tagtgcagtg agtaatgtat ctagagaagg     2460
ggtcttaaac ccagatgcct agactggcga gagaaaataa attatcttat gcaagctgag     2520
cctaaacaaa tggtggaagt tactacgatg gataagtgag cattaagttc atctgaaagg     2580
agaatttgat acccaaatcc aacccatttc tctaggagaa tgagcaccca gtttctgaga     2640
tgtcttcgtt tctcaggaaa atccagaaat cctgggtttt atgtgaaact taatttataa     2700
gtgttgctaa ctaattaaaa ttcaaaaatg tctcagcagc gtggtttcag ccattgtaga     2760
cggtttcagc tttttcacac tggggtgtgt gtgtgtatgg gtgaaaacct ggaagctggc     2820
tgagctgtat cctcttgtac ttcctcctat cattggtgtt tttttgtttg tttgagatgg     2880
agtttcactc ttgttgccca ggtggagtgc aatggcacaa tctcggctca ctgcaacctc     2940
tgcctccggg ttcaagccat tctcctgcct cagcctccca agtagctggg actacaggtg     2960
cccgtcccca cgcccagcta attttttgta tttttagtag agacggggtt ttgccatgtt     3060
```

ggccaggctg gtcttgaact cctgacctca gatccaccca cttcggcctc ccaaagtgct 3120

gggaatacag gcgtgagcca ccgtggccgg ccctcattgg ttcttttaaa ctgtactgca 3180

cttaaatata taattctcct aaacactact tttgactttt ttttagcttt atgtgcattt 3240

cttgtctccc agacttagca acagagaccc tgtctccttt ttaagaaaca ttattgagct 3300

tctactatat gtcaagaact gtggtaggca ctgtagggtc aaaaggatgg ttaagagaat 3360

attcctgact tctaagggcc cagggagatt atataatata ataagattat aggtattata 3420

gaggtattct taaaatgttg agggggtaca gatgaaaaca agatgtattc ttcctgggga 3480

gttggaaagg tttcagaaag atcatatttg agctagacct ttatttgagc agttcagaca 3540

aggataggtt tttattcacc tttgtatcac ccaaggtaag tgctgagata acaataagag 3600

catgatcttg gaaatcagac ctgattttaa attcttgctc tgccacttac ttgacagcag 3660

tcctagaacc tattgtttag catctttgag ccttcaaaat ggtggtaaca tcagctatga 3720

agactggatg aggaaaagta tattataaag catttcaagc agtgcttggc acacacaatt 3780

catcaaattc aacatgctat tcattgtaag acgcatcatt attttatcta ctactaaaga 3840

ggaaaaaata actaattaaa ccatgacaca ccattgattg taagaagaat cccattcaga 3900

gatgttaaaa tatgaaaaaa tgagaaaata tgaaatatat taataagcag ttaagttatt 3960

cctgtgtaaa tgtttacaac catggactgc taggatacac ttccttttct agggtaatga 4020

tgtcatgaca ctatgcccct tgtttaagaa cagtgctaga agtcaggcgc ggtggctcac 4080

gcctgcaatc ccagcacttt gggaggctga ggcgggcgga tcacgaggtc aggagattga 4140

gaccatcctg gttaacacgg tgaaaccccg tctctactaa aaatacaaaa aaattagctg 4200

gtcgtggtgg tgggcgcctg tagtcccagc tactcgggag 4240

<210> 2
<211> 122
<212> DNA
<213> Artificial Sequence

<220>
<223> bisulfite-treated DNA

<400> 2

ucgtgaagta agaagautga uttucguaaa ggtautgaac gautgggtgt aggtguuagg 60

guatggacgt gaagutggaa ucgcgaaatg agttagttgt gggucgutgg uutuaaggag 120

cg 122

<210> 3
<211> 20
<212> DNA

<213> Artificial Sequence

<220>
<223> primer

<400> 3
ggttagaatt tagttattgg 20

<210> 4
<211> 20
<212> DNA
<213> Artificial Sequence

<220>
<223> primer

<400> 4
rtatacacaa acttctcctc 20

<210> 5
<211> 240
<212> DNA
<213> Homo sapiens

<400> 5

**ggccagaatc cagttattgg gatagtcttg acttctacct cagcccaact tctgtggtcg 60**

**taatattagt ttagcttggg tttgccgtga agtaagaaga ctgacttccg caaaggtact 120**

**gaacgactgg gtgtaggtgc cagggcatgg acgtgaagct ggaaccgcga aatgagttag 180**

**ttgtgggccg ctggcctcaa ggagcgaacg tcagcgcgaa gaggagaagc ttgtgcatac 240**

<210> 6
<211> 10
<212> DNA
<213> Artificial Sequence

<220>
<223> Tag sequence

<400> 6
aggaagagag 10

<210> 7
<211> 31
<212> DNA
<213> Artificial Sequence

<220>
<223> T7 promoter sequence

<400> 7
cagtaatacg actcactata gggagaaggc t 31

<210> 8
<211> 25

<212> DNA
<213> Artificial Sequence

<220>
<223> primer

<400> 8
tcgtgaagta agaagattga ttttc 25

<210> 9
<211> 20
<212> DNA
<213> Artificial Sequence

<220>
<223> primer

<400> 9
cgctccttaa aaccaacgac 20

<210> 10
<211> 33
<212> DNA
<213> Artificial Sequence

<220>
<223> primer

<400> 10
gggatattaa gtggagttat tttggtttta gtt 33

<210> 11
<211> 21
<212> DNA
<213> Artificial Sequence

<220>
<223> primer

<400> 11
ccctcccaac atccttccta a 21

<210> 12
<211> 122
<212> DNA
<213> Homo sapiens

<400> 12

ccgtgaagta agaagactga cttccgcaaa ggtactgaac gactgggtgt aggtgccagg 60

gcatggacgt gaagctggaa ccgcgaaatg agttagttgt gggccgctgg cctcaaggag 120

cg 122

**Description**

TECHNICAL FIELD

The present invention relates to a method for obtaining information on uterine body cancer in a subject. The present invention further relates to in vitro use of a marker and a determination device for obtaining information on uterine body cancer.

BACKGROUND

The uterus includes a cervix that is the entrance of the uterus, and a uterine body that is located behind the cervix. Uterine body cancer arises from the uterine body. Uterine body cancer is also referred to as endometrial cancer because it arises from the endometrium. For screening for uterine body cancer, pathological examination (cytodiagnosis and biopsy), in which cells or tissues are collected from the uterine endometrium and observed on a microscope, is typically performed. In this pathological examination, it is necessary to insert a specially-designed tool into the uterine body to collect cells or tissues and such procedure may cause pain and bleeding. This imposes a large burden on subjects.
For screening for uterine body cancer, measurement of tumor markers in blood is also performed. As the tumor markers, CA125 and CA19-9 are used, for example. However, these tumor markers have insufficient sensitivity in detecting cancer, and are also used for cancers of different types from uterine body cancer. Accordingly, these tumor markers are not sufficient in terms of sensitivity and specificity.
Meanwhile, new methods for diagnosing cancer based on genetic information have been studied in recent years. The methods include, for example, a method based on information on methylation of DNA. In this method, CpG sites (5'-(CG)-3') in base sequences of certain genes are used as markers. Then, information such as the presence or absence of cancer cells is obtained based on the analysis results of the methylation status of the markers, and is used as an index for diagnosis of cancer.
Methods for determining cancer by DNA methylation analysis have been studied and developed for uterine body cancer. For example, Esteller M. et al. discloses that the MLL1 genes are highly methylated in endometrial cancerous tissues although they are not methylated in normal endometrial tissues (see Esteller M. et al., AM. J. Pathol. Vol. 155, P. 1767-1772 (1999)). Furlan D. et al. discloses that three genes of MLH1, CDKN2A, and MGMT are highly methylated in tissues of endometrial and ovarian tumors (see Furlan D. et. al, Clin. Cancer Res. vol. 12, p. 3329-3336 (2006)).
The genes with abnormal methylation in uterine body cancer have been reported as described above. These genes, however, are more or less methylated in cancers of different types from uterine body cancer. If methylation analysis is performed using such genes, which are methylated for multiple cancers, as markers, information obtained from the analysis results may contain information pertaining not only to uterine body cancer but also to other cancers. For example, even if information indicating that cancer cells exist in a sample is obtained from the analysis results, it is difficult to determine whether the cancer cells are derived from uterine body cancer or other cancers.
As described so far, the specificity of markers to uterine body cancer is a critical issue in obtaining information on uterine body cancer by methylation analysis of the markers. Thus, there is a demand for development of novel markers specific to uterine body cancer, which enables to specifically detect cancer cells derived from uterine body cancer.
An object of the present invention is to provide a method for obtaining information on uterine body cancer by identifying such a novel marker and analyzing methylation status of the marker. Another object of the present invention is to provide a kit suitably used for the method.

SUMMARY OF THE INVENTION

The present inventors have identified novel markers which are genetic regions specifically methylated in DNA obtained from cancerous tissues of uterine body cancer. The present inventors have found that cancer cells derived from uterine body cancer can be clearly discriminated from other cells (cells of normal tissues, cells of non-cancerous tissues, and cancer cells derived from cancers of different types from uterine body cancer) based on the result obtained by analyzing the methylation status of the markers, thereby completing the present invention.
(1) The present invention provides a method for obtaining information on uterine body cancer including the steps of: preparing a DNA sample from a biological sample collected from a subject; analyzing methylation status of a CpG site located in a promoter region of TMCO1 gene in the DNA sample obtained in the preparing step; and obtaining information on uterine body cancer in the subject based on an analysis result obtained in the analyzing step.
(2) The method according to (1), wherein the analyzing step is a step of analyzing the presence or absence of methylation of at least one CpG site.

(3) The method according to (2), wherein the information on uterine body cancer in the subject is the presence or absence of a cancer cell derived from uterine body cancer in the biological sample collected from the subject, and the information obtaining step is a step of obtaining information indicating that the biological sample contains a cancer cell derived from uterine body cancer when the analysis result indicates the presence of a methylated CpG site.
(4) The method according to (1) to (3), wherein the analyzing step is a step of analyzing methylation frequency.
(5) The method according to (4), wherein the information on uterine body cancer in the subject is the presence or absence of a cancer cell derived from uterine body cancer in the biological sample collected from the subject, and the information obtaining step is a step of obtaining information indicating that the biological sample contains a cancer cell derived from uterine body cancer when the methylation frequency obtained in the analyzing step is higher than a predetermined threshold.
(6) The method according to (1) to (5), wherein the analysis result is obtained by using a kit for obtaining information on uterine body cancer comprising a primer set for analyzing methylation status of at least one CpG site selected from CpG sites located in a promoter region of TMCO1 gene.
(7) The method according to (6), wherein the primer set is a primer set for analyzing the methylation status of the CpG site by at least one method selected from mass spectrometry and methylation-specific PCR method.
(8) The method according to (7), wherein the primer set is at least one selected from a primer set of primers respectively having base sequences SEQ ID NOs: 3 and 4 and a primer set of primers respectively having base sequences SEQ ID NOs: 8 and 9.
(9) The present invention provides in vitro use of a marker for obtaining information on uterine body cancer by methylation analysis, which is at least one CpG site selected from CpG sites located in a promoter region of TMCO1 gene.
(10) The in vitro use of the marker according to (9), wherein the marker is a polynucleotide obtained by subjecting an isolated DNA to bisulfite treatment, the isolated DNA having a contiguous base sequence in an entire or partial promoter region of TMCO1 gene and containing at least one CpG site in the promoter region and at least one cytosine not included in CpG sites.
(11) The in vitro use of the marker according to (10), wherein the marker is a polynucleotide having a base sequence SEQ ID NO: 2.
(12) The present invention provides a determination device comprising: a computer system including a computer containing a processor and a memory controlled by the processor, wherein the memory stores a computer program for enabling the computer to carry out a process including the steps of: obtaining an analysis result on methylation status of a CpG site located in a promoter region of TMCO1 gene in a DNA sample derived from a subject; and outputting a determination result as information on uterine body cancer in the subject based on the resulting analysis result.
(13) The determination device according to (12), wherein the analysis result is presence or absence of methylation of at least one CpG site.
(14) The determination device according to (13), wherein the information on uterine body cancer in the subject is presence or absence of a cancer cell derived from uterine body cancer in the biological sample collected from the subject, and the step of outputting information is the step of outputting information indicating that the biological sample contains a cancer cell derived from uterine body cancer when the analysis result indicates the presence of a methylated CpG site.
(15) The determination device according to (12) to (14) further comprises a measurement device which measures a DNA sample from a biological sample collected from a subject, wherein the measurement device is connected to the computer system.

BRIEF DESCRIPTION OF THE DRAWINGS

Fig. 1 is a graph illustrating a methylation positive rate in a promoter region of TMCO1 gene calculated from methylation data of cancerous tissues of uterine body cancer and normal uterine tissues;
Fig. 2 is a graph illustrating a methylation positive rate in a promoter region of TMCO1 gene calculated from methylation data of various clinical specimens;
Fig. 3A is a graph illustrating a methylation positive rate in a promoter region of a known marker gene MLH1 calculated from methylation data of various clinical specimens;
Fig. 3B is a graph illustrating a methylation positive rate in a promoter region of a known marker gene CDKN2A calculated from methylation data of various clinical specimens;
Fig. 4 is a graph illustrating a methylation score in a promoter region of TMCO1 gene in DNA extracted from normal uterine tissues and cancerous tissues of uterine body cancer;

Fig. 5A is a bar graph illustrating the band intensity of amplified products generated by amplifying DNAs extracted from peripheral blood of healthy subjects and patients with uterine body cancer by a methylation-specific PCR (MSP) method using a primer set of TMCO1 (SEQ ID NOs: 8 and 9);

Fig. 5B is a bar graph illustrating the band intensity of amplified products generated by amplifying DNAs extracted from peripheral blood of healthy subjects and patients with uterine body cancer by the MSP method using a primer set for quality control (SEQ ID NOs: 10 and 11);

Fig. 6 is a schematic view illustrating one example of a determination device for providing information on uterine body cancer in a subject;

Fig. 7 is a block diagram illustrating the functionality configuration of the determination device of Fig. 6;

Fig. 8 is a block diagram illustrating the hardware configuration of the determination device illustrated in Fig. 6; and

Fig. 9 is a flow chart of determination for providing information on uterine body cancer in a subject using the determination device illustrated in Fig. 6.

DETAILED DESCRIPTION OF THE PREFERRED EMBODIMENTS

In the method for obtaining information on uterine body cancer of an embodiment (hereinafter also merely referred to as "method"), a DNA sample is first prepared from a biological sample collected from a subject.

In the embodiment, the biological sample is not particularly limited as long as it is a biological sample containing DNA of a subject, but is preferably a sample containing a genomic DNA such as a clinical specimen. Examples of the clinical specimen include body fluid, urine, and tissues obtained by operations or biopsies. Examples of the body fluid include blood, serum, plasma, lymph fluid, ascitic fluid, bone marrow fluid, and nipple discharge. The biological sample may also be a culture obtained by culturing cells or tissues collected from a subject. Further, the biological sample may be a formalin-fixed paraffin-embedded tissue sample collected from a subject.

The DNA sample can be prepared by extracting DNA from the biological sample. A method for extracting DNA from a biological sample is well-known in the art. DNA can be extracted by, for example, mixing the biological sample with a treatment solution containing a surfactant for solubilization of cells or tissues (such as sodium cholate and sodium dodecyl sulfate) and subjecting the resulting mixture to physical procedure (such as stirring, homogenization, and ultrasonication) to release DNA contained in the biological sample into the mixture. In this case, a supernatant containing DNA released by centrifuging the mixture to precipitate cell debris is preferably used in a later-described analyzing step. The obtained supernatant may be purified by any well-known method in the art. DNA can also be extracted from the biological sample and purified by using a commercially-available kit.

Preferably, the above-described preparing step further comprises a step of fragmenting the extracted DNA. By fragmenting the DNA to have appropriate length, methylated DNA immunoprecipitation (MeDIP) and non-methylated cytosine conversion as described below can be effectively performed.

Fragmentation of DNA may be performed by ultrasonication, alkaline treatment, restriction enzyme treatment, or the like. When DNA is fragmented by alkaline treatment, for example, a sodium hydroxide solution is added to a DNA solution to obtain a final concentration of 0.1 to 1.0N and the mixture is incubated at 10 to 40°C for 5 to 15 minutes to fragment the DNA. When DNA is fragmented by the restriction enzyme treatment, the restriction enzyme is appropriately selected based on the base sequence of DNA, which may be MseI or BamHI, for example.

In the method of the embodiment, the methylation status of a CpG site in a promoter region of TMCO1 gene in the DNA obtained in the preparing step is analyzed.

The term "CpG site" used herein means a site of a sequence in which cytosine (C) and guanine (G) are adjacent in this order from 5' to 3' in the base sequence. The letter "p" in "CpG" represents a phosphodiester bond between cytosine and guanine.

As used herein, "analyzing the methylation status" means analyzing the presence or absence of methylation of a CpG site located in a promoter region of TMCO1 gene or analyzing methylation frequency in the promoter region.

TMCO1 gene is also known as HP10122, PCIA3, PNAS-136, RP11-466F5.7, or TMCC4 in the art. The base sequence in the promoter region of TMCO1 gene is well-known in the art. The base sequence can be obtained from a well-known database provided by, for example, the National Center for Biotechnology Information (NCBI) (http://www.ncbi.nlm.nih.gov/). The ID number of TMCO1 gene is shown in Table 1. The base sequence in the promoter region of TMCO1 gene is represented by SEQ ID NO. 1.

[Table 1]

| Gene symbol | Unigene ID | Entrez Gene ID | SEQ ID. NO. |
|---|---|---|---|
| TMC01 | Hs. 31498 | 54499 | 1 |

In the embodiment, the analyzing step may be a step of analyzing the presence or absence of methylation of at least one CpG site among CpG sites located in a promoter region of TMCO1 gene. The term "presence or absence of methylation" means whether or not cytosine in a CpG site located in the promoter region is methylated. In the embodiment, only one CpG site may be analyzed, but a plurality of CpG sites is preferably analyzed for the presence or absence of methylation.

In another embodiment, the analyzing step may be a step of analyzing methylation frequency in a promoter region of TMCO1 gene. The term "methylation frequency" means a ratio of the number of methylated CpG sites relative to the number of CpG sites located in the promoter region. In this embodiment, a target for analysis may be the entire promoter region or a part of the promoter region including at least one CpG site. The target for analysis may contain only one CpG site, but the target for analysis preferably contains a plurality of CpG sites. The positions and number of CpG sites located in the promoter region of TMCO1 gene are already known, and thus, in the embodiment, the number of methylated CpG sites itself in the promoter region can be used as the methylation frequency.

The methylation frequency may be a "methylation score" obtained by analyzing methylation status of a CpG site in DNA with mass spectrometry such as MassARRAY® as described below. MassARRAY® allows calculation of a methylation score based on a ratio between the area of a peak derived from methylated DNA fragment and the area of a peak derived from non-methylated DNA fragment obtained through measurement of DNA fragments.

In the embodiment, the methylation frequency in the promotor region of TMCO1 gene may be calculated by a hand method or a machine such as a computer.

In the embodiment, the target for analysis may is not particularly limited, and may be any CpG sites (or certain regions including the CpG sites) in the promoter region of TMCO1 gene. The target for analysis may be appropriately selected by a person skilled in the art. The positions and number of CpG sites located in the promoter region of TMCO1 gene are already known. Thus, the target CpG sites or regions may be selected by routine experiments according to the well-known analysis method described below.

Various methods for analyzing methylation status are well-known in the art. The analysis method to be used in the embodiment is not particularly limited, but preferably includes a step of differentiating methylated DNA from non-methylated DNA, a step of amplifying DNA, and a step of detecting methylated DNA and/or non-methylated DNA.

The step of differentiating methylated DNA from non-methylated DNA may include a step of performing methylation sensitive restriction enzyme treatment, a MeDIP method, non-methylated cytosine converting treatment, or the like.

The step of amplifying DNA may include a step of performing PCR, quantitative PCR, IVT (in vitro transcription) amplification, SPIA (trademark) amplification methods, or the like.

The step of detecting methylated DNA and/or non-methylated DNA may include a step of performing electrophoresis, sequence analysis, microarray analysis, mass spectrometry, Southern hybridization, or the like.

The MeDIP method is used to enrich for methylated DNA in a biological sample by immunoprecipitation using an anti-methylated cytosine antibody or an anti-methylated cytidine antibody, or an antibody which specifically recognizes a methylated DNA-binding protein. In the embodiment, the analyzing step may be a step of enriching for methylated DNA in DNA obtained in the extracting step by the MeDIP method and analyzing methylation status of the obtained methylated DNA. The methylated DNA enriched by the MeDIP method may be amplified by, for example, IVT amplification, and the methylation status of the obtained amplified product may be analyzed by using a microarray. This analysis method is referred to as "MeDIP on chip."

The non-methylated cytosine converting treatment is used to react DNA extracted from a biological sample with a non-methylated cytosine conversion agent so as to convert non-methylated cytosine in the DNA to a different base (uracil, thymine, adenine or guanine). The non-methylated cytosine conversion agent is a substance that can react with DNA and convert non-methylated cytosine in the DNA to a different base (uracil, thymine, adenine or guanine). The non-methylated cytosine conversion agent may be, for example, bisulfite such as sodium, potassium, calcium or magnesium bisulfite.

In the treatment using bisulfite, non-methylated cytosine in DNA is converted to uracil due to deamination reaction, while methylated cytosine does not undergo such a base conversion. Thus, the difference in methylation status of a CpG site in DNA is converted to the difference in a base sequence (C and U) by the non-methylated cytosine converting treatment using bisulfite. The non-methylated cytosine converting treatment using bisulfite is referred to as "bisulfite treatment."

When the bisulfite treatment is performed, the additive amount (concentration) of bisulfite is not specifically limited as long as it can sufficiently convert non-methylated cytosine in DNA. For example, the final concentration in a solution containing DNA is 1M or higher, preferably 1M to 15M, and more preferably 3M to 10M. The incubation condition (temperature and time) after addition of bisulfite may be appropriately selected depending on the additive amount of bisulfite. For example, when bisulfite is added at a final concentration of 6M, the incubation is carried out at 50 to 80°C for 10 to 90 minutes.

Methylation status of CpG sites in DNA can be analyzed by analyzing the sequence of DNA after bisulfite treatment and detecting the difference in base sequence from the original sequence. This method is referred to as "bisulfite sequencing."

The methylation status of CpG sites can be alternatively analyzed by mass spectrometry. Specifically, DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is cleaved with RNase A, and the difference in mass (16 Da) due to difference between G and A in the obtained digested fragments is detected using a MALDI-TOF (matrix assisted laser desorption/ionization time-of-flight) mass spectrometer to analyze methylation status of the DNA. This method is referred to as "MassARRAY® analysis."

It is known that the site of IVT product cleaved with RNase A is between an arbitrary base sequence and the adjacent uracil (U) or thymine (T). Thus, the base sequence and mass of the IVT product cleaved with RNase A can be predicted based on the base sequence of the template DNA. Accordingly, it is possible to identify a portion of the base sequence of the template DNA from which each peak obtained in MassARRAY® is originated. For example, when one CpG site is methylated in a DNA fragment, a peak obtained in MassARRAY® shifts to the side with an increased mass for 16 Da. In analysis of a DNA fragment containing plural CpG sites, for example, a shift of 32 Da is shown when two CpG sites are methylated, and a shift of 48 Da is shown when three methylated CpG sites are methylated.

In mass spectrometry such as MassARRAY®, the methylation score of the analyzed DNA fragment can be calculated. For example, when the ratio between the area of the peak of the non-methylated DNA fragment and the area of the peak of the methylated DNA fragment in a chart obtained from the analysis of a DNA fragment having a certain sequence is 1 : 3, the methylation score of the DNA fragment is 0.75 (= 3/(1 + 3)). The methylation score is theoretically 1 when all CpG sites are methylated, and 0 when not all CpG sites are methylated.

The methylation status of CpG sites can be analyzed by a methylation-specific PCR (MSP) method. The MSP method is a method of analyzing the methylation status of CpG sites (the presence or absence of methylation) by amplifying DNA after bisulfite treatment by PCR using a primer set described below and determining the presence or absence of a PCR product.

The MSP method utilizes a primer set that can amplify a base sequence where a CpG site to be analyzed is methylated (i.e. cytosine is not converted to uracil), but cannot amplify a base sequence where a CpG site is not methylated (i.e. cytosine is converted to uracil). According to the MSP method using such a primer set, the presence of a PCR product indicates the methylation of the CpG site to be analyzed.

The MSP method may also utilize a primer set that cannot amplify a base sequence where cytosine in a CpG site to be analyzed is not converted to uracil, but can amplify a base sequence where cytosine in a CpG site is converted to uracil. In this case, the absence of a PCR product indicates the methylation of the CpG site to be analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end of the primer or in the vicinity thereof.

The methylation status of CpG sites may be alternatively analyzed with a microarray. In this case, the microarray for analysis may be prepared by immobilizing a nucleic probe complementary to the base sequence in a promoter region of TMCO1 gene on a substrate. The microarray can be prepared according to a well-known method in the art.

In the analysis using a microarray, DNA extracted from a biological sample is preferably labeled with a labeling substance well-known in the art. Thus, the determination method of the embodiment preferably further includes a step of labeling the extracted DNA. The labeling step is advantageously carried out after the DNA amplifying step because all DNA in the biological sample can be labeled. Examples of the labeling substance include fluorescent substances, haptens such as biotin, and radioactive substances. Examples of the fluorescent substances include Cy3, Cy5, FITC, and Alexa Fluor™. Labeling of DNA facilitates measurement of a signal from a probe on the microarray. The method for labeling DNA with the labeling substance is well-known in the art.

The above signal may be any suitable signal depending on the type of microarrays. For example, the signal may be an electric signal generated when a DNA fragment hybridizes to a probe on the microarray, or a fluorescence or luminescence signal generated from a labeling substance when DNA to be analyzed is labeled as described above. The signal can be detected using a scanner included in a normal microarray analyzer. Examples of the scanner include GeneChip® Scanner3000 7G (Affymetrix, Inc.), and Illumina® BeadArray Reader (Illumina, Inc.).

In the method of the embodiment, information on uterine body cancer in a subject is obtained based on the analysis result obtained in the analyzing step. The information on uterine body cancer is not particularly limited as long as it may be an index on diagnosis of uterine body cancer or may be used as an auxiliary tool for diagnosis of uterine body cancer, and is preferably information indicative of occurrence or status of uterine body cancer or both of them in a subject. The information may include, for example, the presence or absence of cancer cells derived from uterine body cancer in a biological sample collected from a subject, the possibility of occurrence of uterine body cancer in a subject, or the risk for future occurrence of uterine body cancer in a subject. The information on uterine body cancer in a subject who has already been affected by uterine body cancer may include prognosis of the subject, or a degree of progression (stage). The information on uterine body cancer based on the analysis result obtained in the analyzing step does not substantially contain information on other cancers of different types from uterine body cancer. This is because the promoter region used as the marker in the method of the embodiment is methylated specifically to cancer cells derived from uterine body cancer.

In the embodiment, when the analysis result in the analyzing step indicates the presence of methylated CpG sites, information indicating the occurrence of uterine body cancer or indicating that the status of uterine body cancer is poor (or aggravated) can be obtained.

In another embodiment, such information can be obtained when the methylation frequency obtained in the analyzing step is higher than or equal to a certain threshold.

More specifically, the information may be indicative of the presence of cancer cells derived from uterine body in a biological sample. The information may alternatively indicate that a subject has a high risk for being affected by uterine body cancer or that a subject has already been affected by uterine body cancer. For a subject who has already been affected by uterine body cancer, the information may indicate that prognosis of the subject is poor (or aggravated) or that the cancer is in a progressed stage.

In contrast, when the result in the analyzing step indicates the absence of methylated CpG sites, information suggesting no occurrence of uterine body cancer or information indicating that uterine body cancer is in a preferable status can be obtained. Alternatively, such information can be obtained when the methylation frequency obtained in the analyzing step is lower than a certain threshold. More specifically, the information may be indicative of the absence of cancer cells derived from uterine body cancer in a biological sample. The information may alternatively indicate that a subject has a low risk for being affected by uterine body cancer or that a subject has not been affected by uterine body cancer. For a subject who has already been affected by uterine body cancer, the information may be indicative of a preferable prognosis of the subject or indicate that the cancer is in a relatively early stage.

The threshold is not particularly limited and may be empirically set based on accumulated data on various biological samples. The threshold may be alternatively set as follows. First, methylation frequency is analyzed for DNA extracted from a biological sample which is confirmed to be devoid of cancer cells derived from uterine body cancer (normal uterine tissues or cells) and a biological sample containing a cancer cell derived from uterine body cancer. Next, based on the obtained analysis results, a threshold is set within a range that is higher than the methylation frequency of the biological sample devoid of cancer cells and lower than the methylation frequency of the biological sample containing the cancer cell. Preferably, the threshold is set as a value that can highly accurately differentiate between the biological sample devoid of cancer cells and the biological sample containing the cancer cell.

The scope of the present invention also encompasses a marker for obtaining information on uterine body cancer by methylation analysis (also simply referred to as "marker"). The marker of the embodiment is at least one CpG site selected from CpG sites located in a promoter region of TMCO1 gene. In the embodiment, the methylation status of the marker in a DNA sample prepared from a biological sample collected from a subject may be analyzed, and information on uterine body cancer in the subject can be obtained based on the analysis result. The analysis of methylation status and the obtainment of information on uterine body cancer are the same as previously described.

The scope of the present invention encompasses the use of a polynucleotide obtained by subjecting an isolated DNA to bisulfite treatment, in which the isolated DNA has a contiguous base sequence in the entire or partial promoter region of TMCO1 gene and contains at least one CpG site in the promoter region and at least one cytosine not included in CpG sites (also simply referred to as "polynucleotide"), as a marker for obtaining information on uterine body cancer. The term "cytosine not included in CpG sites" may be any cytosine other than those contained in CpG sites and may include, for example, cytosine in a base sequence in which cytosine (C), and adenine (A), thymine (T) or cytosine (C) are adjacent in this order from 5' to 3' (namely CA, CT or CC).

Regarding the polynucleotide of the embodiment, a non-methylated cytosine in the isolated DNA is converted to uracil by bisulfate treatment of the isolated DNA, while a methylated cytosine is not converted. In the embodiment, the information on uterine body cancer can be obtained by analyzing methylation status of CpG sites in the polynucleotide. The isolated DNA can be obtained in the same manner as that described for preparation of the DNA sample. The bisulfite treatment, the analysis of methylation status and the obtainment of information on uterine body cancer are also the same as previously described.

The size of the polynucleotide of the embodiment is not particularly limited as long as it allows analysis of methylation status by the MSP method, sequencing or mass spectrometry, but is preferably 70 or more and 400 or less bases and more preferably 70 or more and 200 or less bases. Examples of the polynucleotide of the embodiment include a polynucleotide having a base sequence SEQ ID NO: 2. The polynucleotide having the base sequence SEQ ID NO: 2 is suitable for analysis of methylation status by the MSP method.

The scope of the present invention encompasses a kit for obtaining information on uterine body cancer (also simply referred to as "kit"). The kit of the embodiment includes a primer set for analysis of methylation status of at least one CpG site selected from CpG sites located in a promoter region of TMCO1 gene.

In the embodiment, the primer set included in the kit may be any primer set for analysis of methylation status of CpG sites according to mass spectrometry such as MassARRAY® or an analysis method involving PCR amplification such as the MSP method and the bisulfite sequencing method, but is preferably a primer set used for mass spectrometry such as Mass ARRAY® or for the MSP. The base sequence of each primer in the primer set may be appropriately selected by a person skilled in the art based on the base sequence in the promoter region. Examples of the primer set include a primer set of primers respectively having base sequences SEQ ID NOs: 3 and 4 and a primer set of primers respectively having base sequences SEQ ID NOs: 8 and 9.

The scope of the present invention also encompasses a system suitable for providing information on uterine body cancer in a subject. The system may be as follows, for example.

A system suitable for providing information on uterine body cancer in a subject includes a computer containing a processor and a memory controlled by the processor, wherein
the memory stores a computer program for enabling the computer to carry out a process including the steps of:

obtaining an analysis result on methylation status of a CpG site located in a promoter region of TMCO1 gene in a DNA sample derived from a subject; and
providing information on uterine body cancer in the subject based on the resulting analysis result.

The scope of the present invention also encompasses a computer program product for enabling a computer to provide information on uterine body cancer in a subject. The computer program product may be as follows, for example.

A computer program product for enabling a computer to provide information on uterine body cancer in a subject includes a computer readable medium, wherein
the medium includes a computer program for enabling the computer to carry out a process including the steps of:

obtaining an analysis result on methylation status of a CpG site located in a promoter region of TMCO1 gene in a DNA sample derived from a subject; and
providing information on uterine body cancer in the subject based on the resulting analysis result.

Hereinafter, an embodiment of a suitable device for carrying out the method of the embodiment will be described with reference to the drawings. However, the present invention is not limited to this embodiment. Fig. 6 is a schematic view of an example of a determination device for providing information on uterine body cancer in a subject. A determination device 1 illustrated in Fig. 6 includes a measurement device 2 and a computer system 3 connected to the measurement device 2.

In the embodiment, the measurement device 2 is a MALDI-TOF mass spectrometer. The measurement device 2 obtains mass spectrometric information such as the time of flight or the mass-to-charge ratio (m/z value) of a substance to be analyzed. The measurement device 2, onto which a measurement sample prepared from a DNA sample derived from a subject is mounted, obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2, onto which a gel obtained by electrophoresis of a reaction solution after nucleic acid amplification by the MSP method is mounted, detects amplification products. The measurement device 2 then obtains the band intensity data of the amplification products and sends the obtained data to the computer system 3.

The computer system 3 includes a computer main body 3a, an input device 3b, and a display unit 3c for displaying sample information, determination results and the like. The computer system 3 receives the mass spectrometric information from the measurement device 2. The processor in the computer system 3 executes, based on the mass spectrometric information, a program for providing information on uterine body cancer in a subject.

Fig. 7 is a block diagram illustrating the functionality configuration of the determination device of Fig. 6. As illustrated in Fig. 7, the computer system 3 includes an acquisition unit 301, a storage unit 302, a calculation unit 303, a determination unit 304, and an output unit 305. The acquisition unit 301 is communicably connected to the measurement device 2 through a network. The calculation unit 303 and the determination unit 304 are included in a control unit 306.

The acquisition unit 301 obtains information from the measurement device 2. The storage unit 302 stores a threshold necessary for determination and a formula for calculating a methylation score. The calculation unit 303 calculates the methylation score from the information obtained at the acquisition unit 301 according to the formula stored in the storage unit 302. The determination unit 304 determines whether or not the methylation score calculated at the calculation unit 303 is lower than the threshold stored at the storage unit 302. The output unit 305 outputs the determination result from the determination unit 304 as information on uterine body cancer in the subject (e.g., the presence or absence of cancer cells derived from uterine body cancer in the biological sample collected from the subject).

Fig. 8 is a block diagram illustrating the hardware configuration of the determination device in Fig. 6. As illustrated in Fig. 8, the computer main body 3a includes a central processing unit (CPU) 30, a read only memory (ROM) 31, a RAM 32, a hard disk 33, an input/output interface 34, a readout device 35, a communication interface 36, and an image output interface 37. The CPU 30, ROM 31, a random access memory (RAM) 32, the hard disk 33, the input/output interface 34, the readout device 35, the communication interface 36, and the image output interface 37 are data-communicably connected via a bus 38.

The CPU 30 can execute a computer program stored in the ROM 31 and a computer program loaded with the RAM 32. When the CPU 30 executes the application program, the functional blocks described above may be executed. Accordingly, the computer system serves as a terminal that is a determination device for providing information on uterine body cancer in a subject.

ROM 31 is configured to include mask ROM, PROM, EPROM, EEPROM, and the like. ROM 31 stores the computer program executed by the CPU 30 and data used for the execution.

ROM 32 is configured to include SRAM, DRAM, and the like. ROM 32 is used for readout of the computer programs stored in ROM 31 and the hard disk 33. ROM 32 is also used as a work area of CPU 30 in executing these computer programs.

The computer programs, such as an operating system and an application program (a computer program for providing information on uterine body cancer in a subject), to be executed by the CPU 30, and data for executing the computer programs are installed on the hard disk 33.

The readout device 35 is configured to include a flexible disk drive, a CD-ROM drive, a DVD-ROM drive, and the like. The readout device 35 can read out the computer program or data stored on a portable recording medium 40.

The input/output interface 34 is configured to include a serial interface such as USB, IEEE1394, and RS-232C, a parallel interface such as SCSI, IDE, and IEEE1284, an analog interface formed by a D/A converter and an A/D converter, and the like. The input/output interface 34 is connected to the input device 3b such as a keyboard and a mouse. A user can input the data into the computer main body 3a by means of the input device 3b.

The communication interface 36 is, for example, an Ethernet® interface. The computer system 3 can send printing data to a printer via the communication interface 36.

The image output interface 37 is connected to the display unit 3c including a LCD, a CRT and the like. Accordingly, the display unit 3c can output an image signal according to image data from the CPU 30. The display unit 3c displays an image (on a screen) according to the input image signal.

Subsequently, the processing procedure performed by the determination device 1 for providing information on uterine body cancer in a subject will be described. Fig. 9 is a flow chart for providing information on uterine body cancer using the determination device of Fig. 6. In an illustrated example, a peak area is calculated based on mass spectrometric information of a nucleic acid in a measurement sample prepared from a DNA sample derived from a subject, and a methylation score is calculated from the obtained peak area, so as to determine whether or not the methylation score is lower than a threshold. However, the present invention is not limited to this embodiment.

In the step S1-1, the acquisition unit 301 in the determination device 1 obtains mass spectrometric information from the measurement device 2. In the step S1-2, the calculation unit 303 calculates a peak area from the mass spectrometric information obtained at the acquisition unit 301 and sends the peak area to the storage unit 302. In the step S1-3, the calculation unit 303 calculates a methylation score based on the peak area stored in the storage unit 302 according to the formula stored in the storage unit 302.

In the step S1-4, the determination unit 304 determines whether or not the methylation score calculated at the calculation unit 303 is lower than the threshold stored in the storage unit 302. When the methylation score is lower than the threshold, the process proceeds to the step S1-5 and the determination unit 304 sends, to the output unit 305, a determination result indicating that the biological sample collected from the subject does not contain cancer cells derived from uterine body cancer. When the methylation score is not lower than the threshold (i.e., the methylation score is the threshold or more), the determination unit 304 sends, to the output unit 305, a determination result indicating that the biological sample collected from the subject contains cancer cells derived from uterine body cancer.

In the step S1-7, the output unit 305 outputs the determination result as information on uterine body cancer in the subject, so that the display unit 3c displays the result and/or the printer prints out the result. Accordingly, the determination device can provide, to a physician or the like, information assisting the physician or the like to judge whether or not the subject has uterine body cancer.

Hereinafter, the present invention will be described in detail with reference to Examples, but the present invention is not limited thereto.

[Examples]

Example 1: Identification of Novel Markers Utilizing Methylation Data of Cancerous Tissues of Uterine Body Cancer and Normal Uterine Tissues

(1) Collection of Methylation Data

In Example 1, methylation data from Infinium, which are published in TCGA (The Cancer Genome Atlas: http://tcga-data.nci.nih.gov/tcga/tcgaHome2.jsp), were collected for cancerous tissues (70 specimens) of uterine body cancer and normal uterine tissues (1 specimens).

(2) Identification of Novel Marker

The Infinium HumanMethylation27 BeadChip includes probes for methylated CpG sites and probes for non-methylated CpG sites of 27,578 CpG sites on human genome. In this example, the signal intensity (signal M) from the probes for methylated CpG sites and the signal intensity (signal U) from the probes for non-methylated CpG sites were detected on BeadArray Reader, and the methylation rate (mCpG) of CpG sites in the gene was calculated according to the following calculation formula:

$$(\text{mCpG}) = (\text{signal M})/\{(\text{signal M}) + (\text{signal U})\}$$

The threshold was set to "0.4" for the obtained methylation rate (mCpG) of each gene. The specimens having their methylation rates of the threshold or higher are referred to as "methylation positive specimens." Based on the number of the methylation positive specimens, the methylation positive rates (%) of the genes in various tissues were calculated by the following formula:

$$\text{Methylation positive rate (\%)} = (\text{the number of methylation positive specimens/the total number of specimens}) \times 100$$

For the cancerous tissue specimens, for example, the methylation positive rate of each gene was calculated by "(the number of methylation positive specimens among cancerous tissue specimens)/the total number of cancerous tissue specimens) × 100. When a statistically significant difference between cancerous tissue specimens and normal uterine tissue specimens was observed, the gene was defined as a marker methylated in cancerous tissues.

As a result of data mining using Infinium HumanMethylation27 BeadChip (Illumina, Inc.), the promoter region of TMCO1 gene was identified as a marker specifically methylated in cancerous tissues of uterine body cancer (see Fig. 1). This marker is also referred to as the present marker hereinbelow.

Example 2: Comparison of Methylation Data Between Cancer/Tumor Tissue Specimens Derived from Plural Types of Cancer/Tumor, Non-Cancerous Tissue Specimens, and Normal Tissue Specimens

(1) Collection of Methylation Data

In Example 2, in addition to cancerous tissues of uterine body cancer (70 specimens) and normal uterine tissue (1 specimen) shown in Example 1, methylation data of 9 types of cancer/tumor tissue specimens, 6 types of non-cancerous tissue specimens, and 13 types of normal tissue specimens were compared. The number of specimens for the respective tissues is shown in the following tables.

[Table 2]

| Cancer/tumor tissue | |
|---|---|
| Tissue | The number of specimens |
| Brain tumor (Brain) | 283 |
| Colon cancer (Colon) | 236 |

(continued)

| Cancer/tumor tissue | |
|---|---|
| Tissue | The number of specimens |
| Breast cancer (Breast) | 186 |
| Lung cancer (Lung) | 59 |
| Gastric cancer (Gastric) | 82 |
| Hepatocellular cancer (Liver) | 100 |
| Renal cell cancer (Kidney) | 219 |
| Ovary | 519 |
| Prostate | 93 |

[Table 3]

| Non-cancerous tissues | |
|---|---|
| Tissues | The number of specimens |
| Colon mucosa (Colon) | 16 |
| Lung | 59 |
| Liver cirrhosis tissue (Liver) | 39 |
| Kidney | 199 |
| Ovary | 16 |
| Prostate | 87 |

[Table 4]

| Normal tissue | |
|---|---|
| Tissues | The number of specimens |
| Normal brain (Brain) | 2 |
| Normal oral mucosa (Oral) | 2 |
| Normal lung (Lung) | 2 |
| Normal colon mucosa (Colon) | 2 |
| Normal liver (Liver) | 2 |
| Normal skeletal muscle (Skeletal) | 2 |
| Normal testis (Testis) | 1 |
| Normal stomach (Stomach) | 2 |
| Normal pancreas (Pancreas) | 1 |
| Normal spleen (Spleen) | 1 |
| Normal kidney (Kidney) | 2 |
| Peripheral blood from healthy subjects (Blood) | 2 |
| Blood cell tissues from healthy subjects (Blood) | 93 |

The methylation data of specimens with cancerous tissues derived from stomach cancer, specimens with cancerous tissues derived from hepatocellular cancer, specimens with non-cancerous tissues derived from hepatocellular cancer, and specimens with normal tissues derived from normal liver, and 12 types of specimens with normal tissues of the above-described specimens were measured by Infinium Methylation Assay using Infinium HumanMethylation27 BeadChip (Illumina, Inc.) in the same manner as in Example 1. The methylation data of the other specimens were obtained from TCGA (The Cancer Genome Atlas: http://tcga-data.nci.nih.gov/tcga/tcgaHome2.jsp). The methylation data of lung cancer tissues, prostate cancer tissues, and blood cell tissues from healthy subjects were obtained using Infinium HumanMethylation27 Bead Chip(Illumina, Inc.), which were published in the following documents.

- Lung cancer: Selamat SA, Chung BS, Girard L, Zhang W et al. Genome-scale analyses of DNA methylation in lung adenocancer and integration with mRNA expression. Genome Res. Jul;22(7):1197-211(2012).
- Prostate cancer: Kobayashi Y, Absher DM, Gulzar ZG, Young SR, McKenney JK, Peehl DM, Brooks JD, Myers RM, Sherlock G. DNA methylation profiling reveals novel biomarkers and important roles for DNA methyltransferases in prostate cancer. Genome Res. Jul 21(7):1017-27(2011)
- Data on blood cell tissues in peripheral blood from 93 healthy subjects: Salhia B, Baker A, Ahmann G, Auclair D, Fonseca R, Carpten J. DNA methylation analyses determines the high frequency of genic hypomethylation and low frequency of hypermethylation events in plasma cell tumors. Cancer Res. Sep 1;70(17):6934-44(2010).

The methylation data described herein are the methylation rate (mCpG) of CpG sites in TMCO1 obtained as follows. The Infinium HumanMethylation27 BeadChip includes probes for methylated CpG sites and probes for non-methylated CpG sites of 27,578 CpG sites on human genome. The signal intensity (signal M) from the probes for methylated CpG sites and the signal intensity (signal U) from the probes for non-methylated CpG sites in the target gene (TMCO1 in this embodiment) were detected on BeadArray Reader, and the methylation rate (mCpG) of CpG sites in the gene was calculated according to the following calculation formula:

$$(\text{mCpG}) = (\text{signal M})/\{(\text{signal M}) + (\text{signal U})\}$$

(2) Comparison of Methylation Positive Rates between Cancer/Tumor Tissue Specimens, Non-Cancerous Tissue Specimens, and Normal Tissue Specimens

The obtained methylation rate (mCpG) was defined as "methylation positive" when a statistically significant difference between tumor tissue specimens and normal tissue specimens was observed. Then, the methylation positive rate (%) for each cancer was calculated according to the following formula:

$$\text{Methylation positive rate (\%)} = (\text{the number of methylation positive specimens/the total number of specimens}) \times 100$$

For example, for brain tumor, the methylation positive rate was calculated by "(the number of methylation positive specimens among the brain tumor tissue specimens/the total number of the brain tumor tissue specimens = 283) × 100."

The results are shown in Fig. 2. In Fig. 2, "normal tissues" represent, among the tissues indicated in Table 4, normal tissues excluding 93 specimens of blood cell tissues from healthy subjects, and "normal blood" represents 93 specimens of blood cell tissue from healthy subjects. As obvious from Fig. 2, the present marker was highly methylated specifically to uterine body cancer.

Comparative Example 1: Comparison of Methylation Positive Rates among Cancer/Tumor Tissue Specimens Derived from Plural Types of Cancer/Tumor, Non-Cancerous Tissue Specimens, and Normal Tissue Specimens

The methylation positive rate was calculated for MLH1 and CDKN2A genes (hereinafter referred to as "known markers") which have already been known to be methylated in cancer cells derived from uterine body cancer, in the same manner as in Example 2, for the respective tissues. The results are shown in Figs. 3A and 3B.

Referring to Fig. 3A, in MLH1, the positive rate in uterine body cancer was not obtained and the methylation for other types of cancers was detected. This marker was thus proven to be ineffective as a marker for diagnosing uterine body cancer. Referring to Fig. 3B, in CDKN2A, the positive rate in uterine body cancer was high, but the methylation was detected in other types of cancers and non-cancerous tissues as well. Accordingly, CDKN2A has low specificity to uterine body cancer. Thus, CDKN2A excels in sensitivity but has low specificity as a maker for uterine body cancer.

DCKN2A has a number of problems with its capability as a marker used for diagnosis of uterine body cancer.

Example 3: Comparison of Methylation Data between Normal Uterus and Uterine Body Cancer (MassARRAY)

(1) Biological Samples

In Example 3, as biological samples, normal uterine tissues (3 specimens) and cancerous tissues of uterine body cancer (3 specimens) were used.

(2) Preparation of Measurement Samples and Control Samples

(i) Extraction of Genomic DNA

Genomic DNA was extracted from the respective tissues with the use of QIAamp DNA Mini Kit (QIAGEN). The genomic DNA contained in an obtained DNA solution was fragmented by Bioruptor (COSMO BIO Co., Ltd.). For preparing calibration curves in mass spectrometry, genomic DNA of human peripheral blood lymphocytes was used as the control genomic DNA. The genomic DNA from human peripheral blood lymphocytes was amplified with the use of GenomiPhi v2DNA Amplification Kit (GE Healthcare Life Sciences). The obtained amplified product consisted of non-methylated DNA. The amplification product was then fragmented with Bioruptor (COSMO BIO Co., Ltd.) to obtain a solution of non-methylated DNA fragments (0% methylated DNA). A portion of the solution of non-methylated DNA fragments was subjected to reaction with SssI methylase (New England Biolabs) to methylate all cytosines in CG sequences, and a solution of methylated DNA fragments (100% methylated DNA) was obtained. Then, the 0% methylated DNA solution and the 100% methylated DNA solution were mixed at a certain proportion to obtain a 25% methylated DNA solution, a 50% methylated DNA solution, and a 75% methylated DNA solution.

(ii) Bisulfite Treatment

The respective DNA fragments (500 ng) obtained as described above were subjected to bisulfite treatment with the use of EZ DNA Methylation Kit (Zymo Research), and the treated genomic DNA was dissolved in sterilized distilled water (80 μl).

(iii) PCR and IVT Amplification

The methylated cytosine and urasil included in the respective bisulfite-treated DNA fragments were converted by the PCR and IVT amplification into guanine and adenine.

Incidentally, it has been confirmed by MassARRAY® analysis using control samples described later that the primer set used in the PCR amplifies the methylated DNA and non-methylated DNA evenly. The sequences of the present primer set are shown as below. The base sequence (plus-strand (TOP) sequence) in the promoter region of TMCO1 gene to be analyzed by a primer set below is shown in SEQ ID NO: 5.

Forward: GGTTAGAATTTAGTTATTGG (SEQ ID NO: 3)
Reverse: RTATACACAAACTTCTCCTC (SEQ ID NO: 4)

(R represents A or G)

The following tag sequence and T7 promoter sequence were added to the 5' ends of the forward and reverse primers of the primer set for IVT reaction.

- Tag sequence: AGGAAGAGAG (SEQ ID NO: 6)
- T7 promoter sequence: CAGTAATACGACTCACTATAGGGAGAAGGCT (SEQ ID NO: 7)

A PCR reaction solution was prepared by mixing the following reagents.

| | |
|---|---|
| 10x Hot Star buffer (QIAGEN) | 0.5 μL |
| 25 mM dNTP mix | 0.04 μL |
| Hot Star Taq (5U/ μL) (QIAGEN) | 0.04 μL |

(continued)

| | |
|---|---|
| Primer mix | 2.0 μL |
| DNA solution | 1.0 μL |
| Water | 1.42 μL |
| Total | 5.0 μL |

The PCT reaction was carried out in the above reaction solution under the following conditions.
1 cycle of 94°C for 15 minutes;
45 cycles of 94°C for 20 seconds, 52°C for 30 seconds, and 72°C for 1 minute; and
72°C for 3 minutes

The obtained PCR products were dephosphorylated by SAP (Shrimp Alkaline Phosphatase) contained in a MassCLEAVE™ Reagent kit (Sequenom, Inc.). Then, the following reaction solution prepared by the kit was added to the PCR products.

| | |
|---|---|
| 5x T7 R&DNA polymerase buffer | 0.89 μL |
| T Cleavage mix | 0.24 μL |
| 100 mM DTT | 0.22 μL |
| T7 R&DNA polymerase | 0.44 μL |
| RNase A | 0.06 μL |
| RNase-free water | 3.15 μL |
| Total | 5.0 μL |

The obtained mixture was incubated at 37°C for three hours to induce the IVT reaction and urasil- or thymine-specific cleavage. The resulting reaction products were purified by Clean Resin (Sequenom, Inc.) to obtain samples for mass spectrometry. These samples, in which samples derived from genomic DNA extracted from the clinical specimens were designated as measurement samples and samples derived from control genomic DNA were designated as control samples, were used for mass spectrometry described later.

(3) Analysis of Methylation Status by Mass Spectrometry using MassARRAY®

(i) Preparation of Calibration Curves

The control samples were subjected to the mass spectrometric analysis twice independently. The calibration curves were prepared for the respective primer sets based on the obtained analysis results, and correlation coefficients were calculated. Accordingly, it was confirmed that the used primer sets amplified the methylated DNA and non-methylated DNA evenly.

(ii) Analysis of Measurement Samples

The obtained measurement samples were subjected to the mass spectrometric analysis to obtain peaks of DNA fragments included in the measurement samples. Then, a portion of the base sequence of TMCO1, from which each obtained peak is originated, is identified. A methylation score is calculated from the ratio between the area of the peaks of the fragments including the methylated CpG sites and the area of peaks of the fragments not including the methylated CpG sites in the fragments originating from the same base sequence. The results are shown in Table 4.

Referring to Fig. 4, the methylation status of the present marker was quantified by the MassARRAY® for normal uterine tissues (3 samples) and cancerous tissues of uterine body cancer (3 samples). The results show that the methylation was hardly detected in the normal uterine tissues, while at least two of the three samples with uterine body cancer were highly methylated. The result further shows that the methylation score of the present marker and uterine body cancer were correlated similarly to the result from the Infinium method of Example 1.

Example 4: Comparison of Methylation Data (MSP) between Plasma from Healthy Subjects and Plasma from Patients with Uterine Body Cancer

In Example 4, peripheral blood collected from healthy subjects and patients with uterine body cancer were used as biological samples, and the methylation analysis on CpG sites in the promoter region of TMO1 was performed by the MSP method.

(1) Biological Samples

In this example, peripheral blood collected from patients with uterine body cancer (7 specimens) were used as biological samples, and peripheral blood collected from healthy subjects (3 specimens) were used as control samples.

(2) Preparation of Measurement Samples

(i) Extraction of Genomic DNA

By a conventional method, plasma was obtained from peripheral blood (2 ml) of each specimen, and genomic DNA was extracted from the obtained plasma with use of QIAamp Circulating Nucleic Acid Kit (QIAGEN).

(ii) Bisulfite Treatment

The respective DNA fragments (500 ng) obtained as described above were subjected to bisulfite treatment with use of EZ DNA Methylation Kit (Zymo Research), and the treated genomic DNA was dissolved in sterilized distilled water (80 μL).

(3) MSP

Qualitative MSP was carried out using the measurement samples obtained in the above section (2). The compositions of the PCR reagent, primer sets, and PCR conditions are shown below.

<PCR reagent>

| | |
|---|---|
| DW (sterilized water) | 10.88 μL |
| 10 × PCR buffer with MgCl2 (Roche) | 1.5 μL |
| 10 mM dNTP mix | 0.3 μL |
| 10μM sense primer | 0.6 μL |
| 10μM antisense primer | 0.6 μL |
| Faststart Taq polymerase (Roche) | 0.12 μL |
| Measurement sample | 1.0 μL |
| Total | 15.0 μL |

<Primer Set>

The base sequence of each primer used for MSP is shown below.

- Primer set for TMCO1

Sense primer: TCGTGAAGTAAGAAGATTGATTTTC (SEQ ID NO: 8)
Antisense primer: CGCTCCTTAAAACCAACGAC (SEQ ID NO: 9)

- Primer set for accuracy control

Sense primer: GGGATATTAAGTGGAGTTATTTTGGTTTTAGTT (SEQ ID NO: 10)
Antisense primer: CCCTCCCAACATCCTTCCTAA (SEQ ID NO: 11)

In MSP using the primer set for TMCO1, an amplified product can be obtained when CpG sites in an amplified region are methylated in DNA after bisulfite treatment. The primer set for accuracy control is used to determine whether the bisulfite treatment is properly performed. A base sequence (plus-strand (TOP) sequence) in the region to be analyzed by the primer set in the promoter region of TMCO1 is shown in SEQ ID NO: 12.

<PCR reaction conditions>

95 °C for 6 minutes;
50 cycles of 95°C for 30 seconds, X°C for 30 seconds, and 72°C for 30 seconds; and
keep at 16°C.

In the above reaction conditions, "X" represents the annealing temperature. The annealing temperature was 54°C for the primer set for TMCO1, and 60°C for the primer set for accuracy control.

(4) Analysis of MSP results

The amplified product obtained by MSP was observed by 2% agarose gel electrophoresis. The band intensity of each amplified product is graphically illustrated (see Figs. 5A and 5B). In Fig. 5A, the bars illustrated as peripheral blood from healthy subjects represent the background, indicating that no band was detected. Referring to Fig. 5A, the MSP results on TMCO1 showed that no band was detected in peripheral blood from healthy subjects, while bands were detected in peripheral blood of 4 patients out of 7 patients with uterine body cancer. As is clear from Fig. 5B, bands were detected in all of the samples by PCR using the primer set for accuracy control. This shows that the bisulfite treatment of the samples was properly performed. It turns out that, even when peripheral blood was used as a biological sample, the present marker was highly methylated for patients with uterine body cancer and the methylation was not detected for healthy subjects. Thus, it turns out that the present marker is highly specific to uterine body cancer and is advantageously useful for determining the presence or absence of cancer cells derived from uterine body cancer in a blood sample.

## Claims

1. A method for obtaining information on uterine body cancer comprising the steps of:

   preparing a DNA sample from a biological sample collected from a subject;
   analyzing methylation status of a CpG site located in a promoter region of TMCO1 gene in the DNA sample obtained in the preparing step; and
   obtaining information on uterine body cancer in the subject based on an analysis result obtained in the analyzing step,
   wherein the base sequence in the promoter region of TMCO1 gene is SEQ ID NO: 1.

2. The method according to claim 1, wherein the analyzing step is a step of analyzing the presence or absence of methylation of at least one CpG site.

3. The method according to claim 2, wherein
   the information on uterine body cancer in the subject is the presence or absence of a cancer cell derived from uterine body cancer in the biological sample collected from the subject, and
   the information obtaining step is a step of obtaining information indicating that the biological sample contains a cancer cell derived from uterine body cancer when the analysis result indicates the presence of a methylated CpG site.

4. The method according to claims 1 to 3, wherein the analyzing step is a step of analyzing methylation frequency.

5. The method according to claim 4, wherein
   the information on uterine body cancer in the subject is the presence or absence of a cancer cell derived from uterine body cancer in the biological sample collected from the subject, and
   the information obtaining step is a step of obtaining information indicating that the biological sample contains a cancer cell derived from uterine body cancer when the methylation frequency obtained in the analyzing step is higher than a predetermined threshold.

6. The method according to claims 1 to 5, wherein the analysis result is obtained by using a kit for obtaining information on uterine body cancer comprising a primer set for analyzing methylation status of at least one CpG site selected from CpG sites located in a promoter region of TMCO1 gene.

7. The method according to claim 6, wherein the primer set is a primer set for analyzing the methylation status of the CpG site by at least one method selected from mass spectrometry and methylation-specific PCR method.

8. The method according to claim 7, wherein the primer set is at least one selected from a primer set of primers respectively having base sequences SEQ ID NOs: 3 and 4 and a primer set of primers respectively having base sequences SEQ ID NOs: 8 and 9.

9. In vitro use of a marker for obtaining information on uterine body cancer by methylation analysis, which marker is at least one CpG site selected from CpG sites located in a promoter region of TMCO1 gene,
   wherein the base sequence in the promoter region of TMCO1 gene is SEQ ID NO: 1.

10. The in vitro use of the marker according to claim 9,
    wherein the marker is a polynucleotide obtained by subjecting an isolated DNA to bisulfite treatment, the isolated DNA having a contiguous base sequence in an entire or partial promoter region of TMCO1 gene and containing at least one CpG site in the promoter region and at least one cytosine not included in CpG sites.

11. The in vitro use of the marker according to claim 10, wherein the marker is a polynucleotide having a base sequence SEQ ID NO: 2.

12. A determination device comprising:

    a computer system including a computer containing a processor and a memory controlled by the processor, wherein
    the memory stores a computer program for enabling the computer to carry out a process including the steps of:

obtaining an analysis result on methylation status of a CpG site located in a promoter region of TMCO1 gene in a DNA sample derived from a subject; and
outputting a determination result as information on uterine body cancer in the subject based on the resulting analysis result,

wherein the base sequence in the promoter region of TMCO1 gene is SEQ ID NO: 1.

**13.** The determination device according to claim 12, wherein the analysis result is presence or absence of methylation of at least one CpG site.

**14.** The determination device according to claim 13, wherein
the information on uterine body cancer in the subject is presence or absence of a cancer cell derived from uterine body cancer in the biological sample collected from the subject, and
the step of outputting information is the step of outputting information indicating that the biological sample contains a cancer cell derived from uterine body cancer when the analysis result indicates the presence of a methylated CpG site.

**15.** The determination device according to claims 12 to 14 further comprises a measurement device which measures a DNA sample from a biological sample collected from a subject, wherein the measurement device is connected to the computer system.

## Patentansprüche

**1.** Verfahren zum Erhalten von Information über Gebärmutterkörperkrebs, umfassend die Schritte des:

Herstellens einer DNS-Probe aus einer von einem Subjekt gewonnenen, biologischen Probe;
Analysierens des Methylierungsstatus von einer CpG-Stelle, die in einer Promotorregion des TMCO1-Gens lokalisiert ist, in der im Herstellungsschritt erhaltenen DNS-Probe; und des
Erhaltens von Information über Gebärmutterkörperkrebs in dem Subjekt, basierend auf einem in dem Analyseschritt erhaltenen Analyseergebnis,
wobei die Basensequenz in der Promotorregion des TMCO1-Gens SEQ ID NO: 1 ist.

**2.** Verfahren gemäß Anspruch 1, wobei der Analyseschritt ein Schritt des Analysierens des Vorliegens oder Nichtvorliegens von Methylierung mindestens einer CpG-Stelle ist.

**3.** Verfahren gemäß Anspruch 2, wobei
die Information über Gebärmutterkörperkrebs in dem Subjekt das Vorliegen oder Nichtvorliegen von einer von Gebärmutterkörperkrebs abgeleiteten Krebszelle in der von dem Subjekt gewonnenen, biologischen Probe ist, und
der Informationserhaltungsschritt ein Schritt des Erhaltens von Information ist, die darauf hindeutet, dass die biologische Probe eine von Gebärmutterkörperkrebs abgeleitete Krebszelle enthält, wenn das Analyseergebnis auf das Vorliegen von einer methylierten CpG-Stelle hindeutet.

**4.** Verfahren gemäß den Ansprüchen 1 bis 3, wobei der Analyseschritt ein Schritt des Analysierens von Methylierungshäufigkeit ist.

**5.** Verfahren gemäß Anspruch 4, wobei
die Information über Gebärmutterkörperkrebs in dem Subjekt das Vorliegen oder Nichtvorliegen von einer von Gebärmutterkörperkrebs abgeleiteten Krebszelle in der von dem Subjekt gewonnenen, biologischen Probe ist, und
der Informationserhaltungsschritt ein Schritt des Erhaltens von Information ist, die darauf hindeutet, dass die biologische Probe eine von Gebärmutterkörperkrebs abgeleitete Krebszelle enthält, wenn die in dem Analyseschritt erhaltene Methylierungshäufigkeit höher als ein vorbestimmter Schwellenwert ist.

**6.** Verfahren gemäß den Ansprüchen 1 bis 5, wobei das Analyseergebnis unter Verwendung eines Kits zum Erhalten von Information über Gebärmutterkörperkrebs erhalten wird, das einen Primersatz zum Analysieren des Methylierungsstatus von mindestens einer CpG-Stelle umfasst, ausgewählt aus CpG-Stellen, die in einer Promotorregion des TMCO1-Gens lokalisiert sind.

**7.** Verfahren gemäß Anspruch 6, wobei der Primersatz ein Primersatz zum Analysieren des Methylierungsstatus der CpG-Stelle durch mindestens ein Verfahren, ausgewählt aus Massenspektrometrie und methylierungsspezifischem PCR-Verfahren, ist.

**8.** Verfahren gemäß Anspruch 7, wobei der Primersatz mindestens einer ist, ausgewählt aus einem Primersatz an Primern, die die Basensequenzen SEQ ID NOs: 3 beziehungsweise 4 haben, und einem Primersatz an Primern, die die Basensequenzen SEQ ID NOs: 8 beziehungsweise 9 haben.

**9.** In vitro-Verwendung eines Markers zum Erhalten von Information über Gebärmutterkörperkrebs durch Methylierungsanalyse, welcher Marker mindestens eine CpG-Stelle ist, ausgewählt aus CpG-Stellen, die in einer Promotorregion des TMCO1-Gens lokalisiert sind,
wobei die Basensequenz in der Promotorregion des TMCO1-Gens SEQ ID NO: 1 ist.

**10.** In vitro-Verwendung des Markers gemäß Anspruch 9, wobei der Marker ein Polynukleotid ist, das durch das Aussetzen einer isolierten DNS einer Bisulfit-Behandlung erhalten wird, wobei die isolierte DNS eine fortlaufende Basensequenz in einer ganzen oder partiellen Promotorregion des TMCO1-Gens hat und mindestens eine CpG-Stelle in der Promotorregion und mindestens ein nicht in CpG-Stellen eingeschlossenes Cytosin enthält.

**11.** In vitro-Verwendung des Markers gemäß Anspruch 10, wobei der Marker ein Polynukleotid ist, das eine Basensequenz SEQ ID NO: 2 hat.

**12.** Bestimmungsvorrichtung, umfassend:

ein Computersystem einschließlich eines Computers, der einen Prozessor und einen durch den Prozessor kontrollierten Speicher enthält, wobei
der Speicher ein Computerprogramm zur Befähigung des Computers, einen Prozess auszuführen, speichert, einschließlich der Schritte des:

Erhaltens eines Analyseergebnisses über den Methylierungsstatus von einer CpG-Stelle, die in einer Promotorregion des TMCO1-Gens lokalisiert ist, in einer von einem Subjekt abgeleiteten DNS-Probe; und des Ausgebens eines Bestimmungsergebnisses als Information über Gebärmutterkörperkrebs in dem Subjekt, basierend auf dem sich ergebenen Analyseergebnis,

wobei die Basensequenz in der Promotorregion des TMCO1-Gens SEQ ID NO: 1 ist.

**13.** Bestimmungsvorrichtung gemäß Anspruch 12, wobei das Analyseergebnis das Vorliegen oder Nichtvorliegen von Methylierung von mindestens einer CpG-Stelle ist.

**14.** Bestimmungsvorrichtung gemäß Anspruch 13, wobei
die Information über Gebärmutterkörperkrebs in dem Subjekt das Vorliegen oder Nichtvorliegen von einer von Gebärmutterkörperkrebs abgeleiteten Krebszelle in der von dem Subjekt gewonnenen, biologischen Probe ist, und der Schritt des Ausgebens von Information der Schritt des Ausgebens von Information ist, die darauf hindeutet, dass die biologische Probe eine von Gebärmutterkörperkrebs abgeleitete Krebszelle enthält, wenn das Analyseergebnis auf das Vorliegen von einer methylierten CpG-Stelle hindeutet.

**15.** Die Bestimmungsvorrichtung gemäß der Ansprüche 12 bis 14 umfasst weiter eine Messvorrichtung, die eine DNS-Probe von einer von einem Subjekt gewonnenen, biologischen Probe misst,
wobei die Messvorrichtung mit dem Computersystem verbunden ist.

## Revendications

**1.** Procédé d'obtention d'informations sur un cancer du corps utérin comprenant les étapes consistant à :

préparer un échantillon d'ADN à partir d'un échantillon biologique prélevé auprès d'un sujet;
analyser l'état de méthylation d'un site CpG situé dans une région promotrice du gène TMCO1 dans l'échantillon d'ADN obtenu dans l'étape de préparation ; et
obtenir des informations sur un cancer du corps utérin chez le sujet sur la base d'un résultat d'analyse obtenu dans l'étape d'analyse,
dans lequel la séquence de bases dans la région promotrice du gène TMOC1 est SEQ ID NO : 1.

2. Procédé selon la revendication 1, dans lequel l'étape d'analyse est une étape d'analyse de la présence ou de l'absence de méthylation d'au moins un site CpG.

3. Procédé selon la revendication 2, dans lequel

les informations sur un cancer du corps utérin chez le sujet sont la présence ou l'absence d'une cellule cancéreuse dérivée d'un cancer du corps utérin dans l'échantillon biologique prélevé auprès du sujet, et
l'étape d'obtention d'informations est une étape d'obtention d'informations indiquant que l'échantillon biologique contient une cellule cancéreuse dérivée d'un cancer du corps utérin lorsque le résultat d'analyse indique la présence d'un site CpG méthylé.

4. Procédé selon les revendications 1 à 3, dans lequel l'étape d'analyse est une étape d'analyse de fréquence de méthylation.

5. Procédé selon la revendication 4, dans lequel

les informations sur un cancer du corps utérin chez le sujet sont la présence ou l'absence d'une cellule cancéreuse dérivée d'un cancer du corps utérin dans l'échantillon biologique prélevé auprès du sujet, et
l'étape d'obtention d'informations est une étape d'obtention d'informations indiquant que l'échantillon biologique contient une cellule cancéreuse dérivée d'un cancer du corps utérin lorsque la fréquence de méthylation obtenue dans l'étape d'analyse est supérieure à un seuil prédéterminé.

6. Procédé selon les revendications 1 à 5, dans lequel le résultat d'analyse est obtenu en utilisant un kit pour obtenir des informations sur un cancer du corps utérin comprenant un jeu d'amorces pour analyser l'état de méthylation d'au moins un site CpG sélectionné parmi des sites CpG situés dans une région promotrice du gène TMCO1.

7. Procédé selon la revendication 6, dans lequel le jeu d'amorces est un jeu d'amorces pour analyser l'état de méthylation du site CpG par au moins un procédé sélectionné parmi la spectrométrie de masse et un procédé de PCR spécifique à la méthylation.

8. Procédé selon la revendication 7, dans lequel le jeu d'amorces est l'un au moins sélectionné parmi un jeu d'amorces constitué par des amorces ayant respectivement des séquences de bases SEQ ID NO : 3 et 4 et un jeu d'amorces constitué par des amorces ayant respectivement des séquences de bases SEQ ID NO : 8 et 9.

9. Utilisation in vitro d'un marqueur pour obtenir des informations sur un cancer du corps utérin par analyse de méthylation, lequel marqueur est au moins un site CpG sélectionné parmi des sites CpG situés dans une région promotrice du gène TMCO1,

dans laquelle la séquence de bases dans la région promotrice du gène TMCO1 est SEQ ID NO : 1.

10. Utilisation in vitro du marqueur selon la revendication 9,
dans laquelle le marqueur est un polynucléotide obtenu en soumettant un ADN isolé à un traitement au bisulfite, l'ADN isolé ayant une séquence de bases contigüe dans une région promotrice entière ou partielle du gène TMC01 et contenant au moins un site CpG dans la région promotrice et au moins une cytosine non incluse dans des sites CpG.

11. Utilisation in vitro du marqueur selon la revendication 10,
dans laquelle le marqueur est un polynucléotide ayant une séquence de bases SEQ ID NO : 2.

12. Dispositif de détermination comprenant :

un système informatique incluant un ordinateur contenant un processeur et une mémoire commandée par le processeur, dans lequel
la mémoire stocke un programme informatique pour permettre à l'ordinateur d'exécuter un processus incluant les étapes consistant à :

obtenir un résultat d'analyse sur un état de méthylation d'un site CpG situé dans une région promotrice du gène TMCO1 dans un échantillon d'ADN dérivé d'un sujet ; et
sortir un résultat de détermination en tant qu'informations sur un cancer du corps utérin chez le sujet sur la base du résultat d'analyse résultant,

dans lequel la séquence de bases dans la région promotrice du gène TMCO1 est SEQ ID NO : 1.

**13.** Dispositif de détermination selon la revendication 12, dans lequel le résultat d'analyse est la présence ou l'absence de méthylation d'au moins un site CpG.

**14.** Dispositif de détermination selon la revendication 13, dans lequel

les informations sur un cancer du corps utérin chez le sujet est la présence ou l'absence d'une cellule cancéreuse dérivée d'un cancer du corps utérin dans l'échantillon biologique prélevé auprès du sujet, et
l'étape de sortie d'informations est l'étape de sortie d'informations indiquant que l'échantillon biologique contient une cellule de cancer dérivée d'un cancer du corps utérin lorsque le résultat d'analyse indique la présence d'un site CpG méthylé.

**15.** Dispositif de détermination selon les revendications 12 à 14 comprenant en outre un dispositif de mesure qui mesure un échantillon d'ADN provenant d'un échantillon biologique prélevé auprès d'un sujet,
dans lequel le dispositif de mesure est connecté au système informatique.

FIG. 1

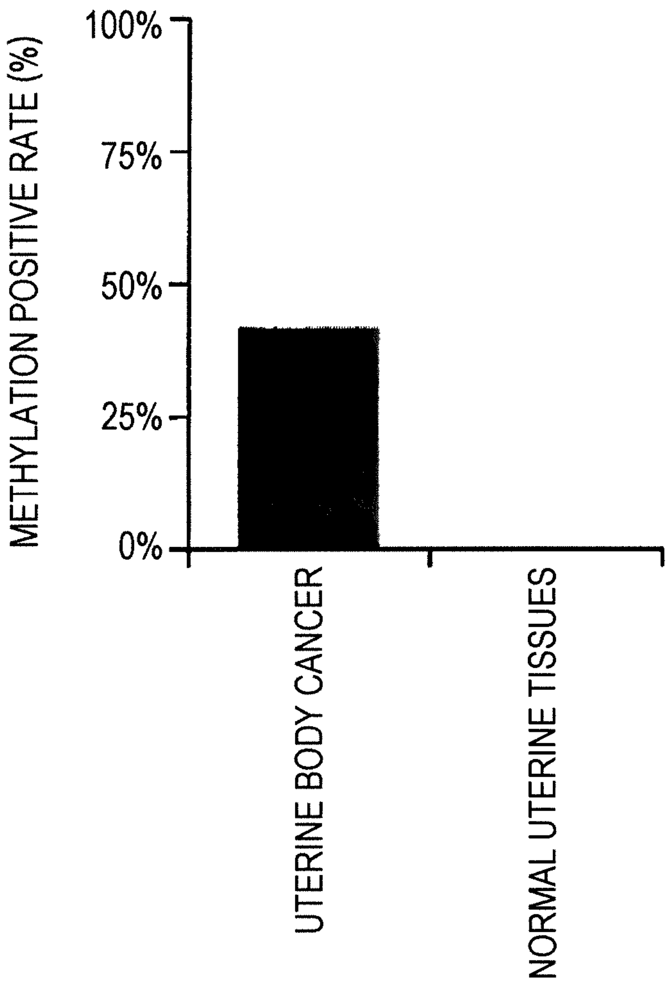
METHYLATION POSITIVE RATE (%)
100%
75%
50%
25%
0%
UTERINE BODY CANCER
NORMAL UTERINE TISSUES

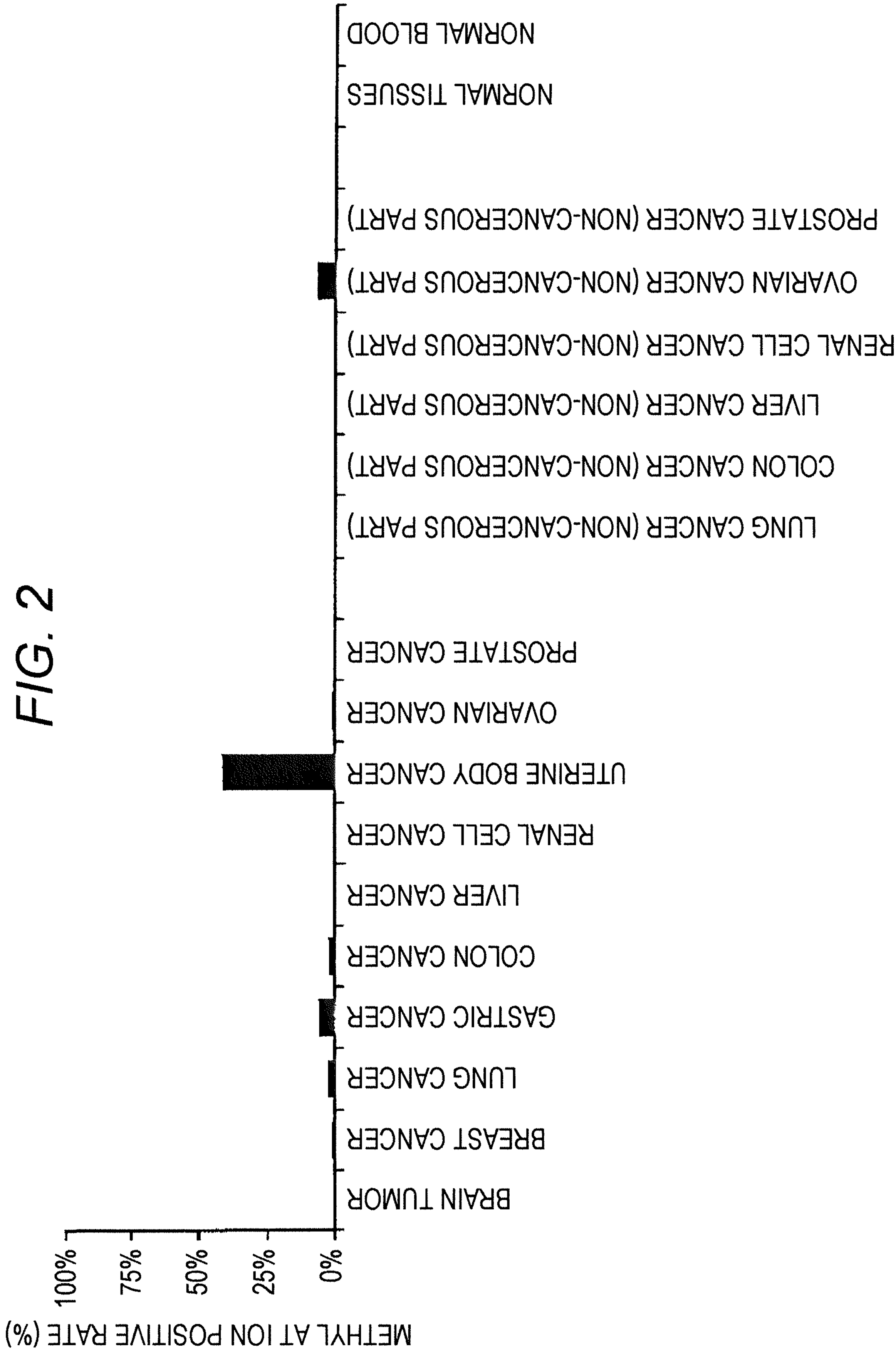
METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CELL CANCER
UTERINE BODY CANCER
OVARIAN CANCER
PROSTATE CANCER
LUNG CANCER (NON-CANCEROUS PART)
COLON CANCER (NON-CANCEROUS PART)
LIVER CANCER (NON-CANCEROUS PART)
RENAL CELL CANCER (NON-CANCEROUS PART)
OVARIAN CANCER (NON-CANCEROUS PART)
PROSTATE CANCER (NON-CANCEROUS PART)
NORMAL TISSUES
NORMAL BLOOD

FIG. 2

*FIG. 3A*

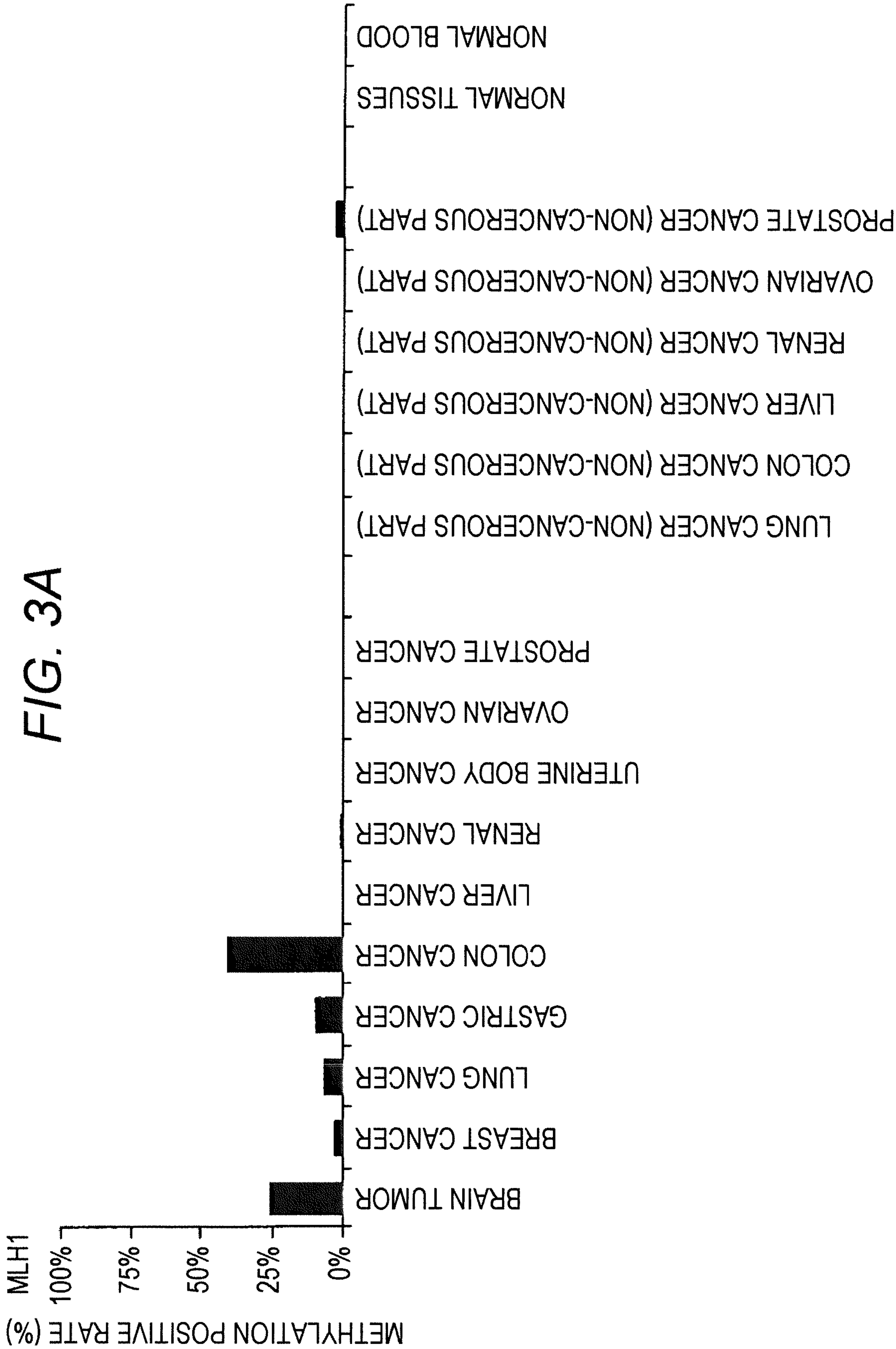

MLH1
METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CANCER
UTERINE BODY CANCER
OVARIAN CANCER
PROSTATE CANCER
LUNG CANCER (NON-CANCEROUS PART)
COLON CANCER (NON-CANCEROUS PART)
LIVER CANCER (NON-CANCEROUS PART)
RENAL CANCER (NON-CANCEROUS PART)
OVARIAN CANCER (NON-CANCEROUS PART)
PROSTATE CANCER (NON-CANCEROUS PART)
NORMAL TISSUES
NORMAL BLOOD

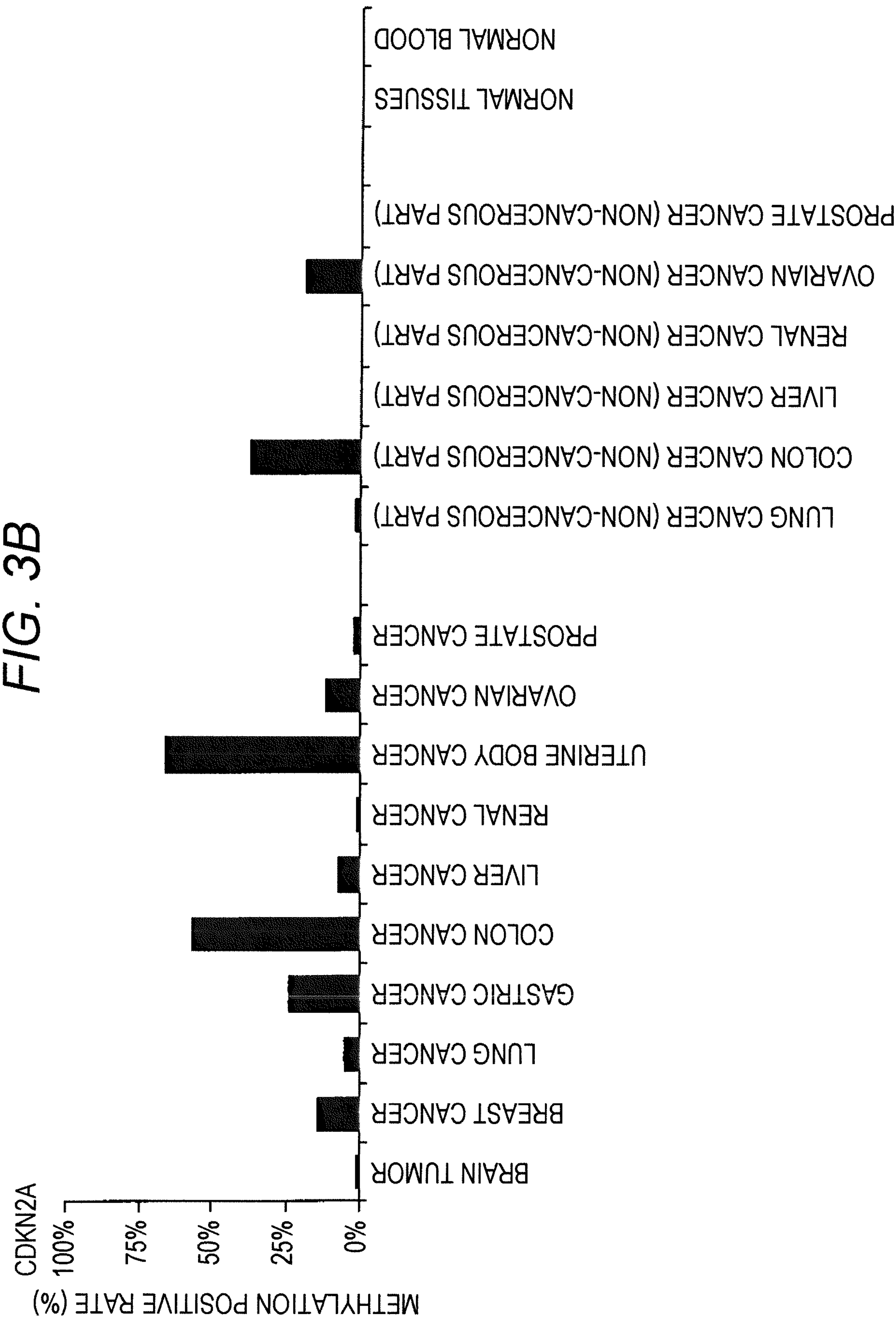
CDKN2A
METHYLATION POSITIVE RATE (%)
100%
75%
50%
25%
0%
BRAIN TUMOR
BREAST CANCER
LUNG CANCER
GASTRIC CANCER
COLON CANCER
LIVER CANCER
RENAL CANCER
UTERINE BODY CANCER
OVARIAN CANCER
PROSTATE CANCER
LUNG CANCER (NON-CANCEROUS PART)
COLON CANCER (NON-CANCEROUS PART)
LIVER CANCER (NON-CANCEROUS PART)
RENAL CANCER (NON-CANCEROUS PART)
OVARIAN CANCER (NON-CANCEROUS PART)
PROSTATE CANCER (NON-CANCEROUS PART)
NORMAL TISSUES
NORMAL BLOOD

FIG. 3B

FIG. 4

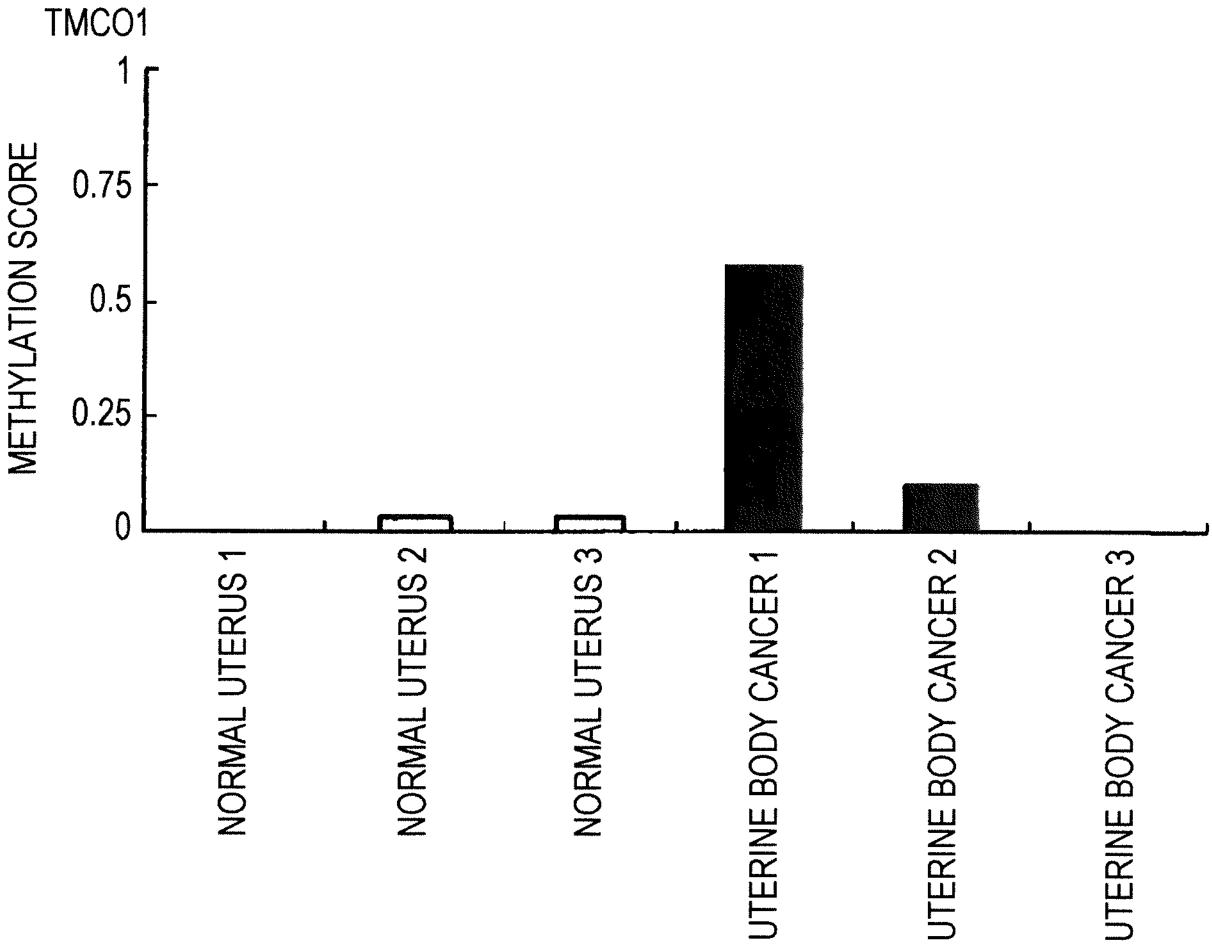
TMCO1
METHYLATION SCORE
1
0.75
0.5
0.25
0
NORMAL UTERUS 1
NORMAL UTERUS 2
NORMAL UTERUS 3
UTERINE BODY CANCER 1
UTERINE BODY CANCER 2
UTERINE BODY CANCER 3

FIG. 5A

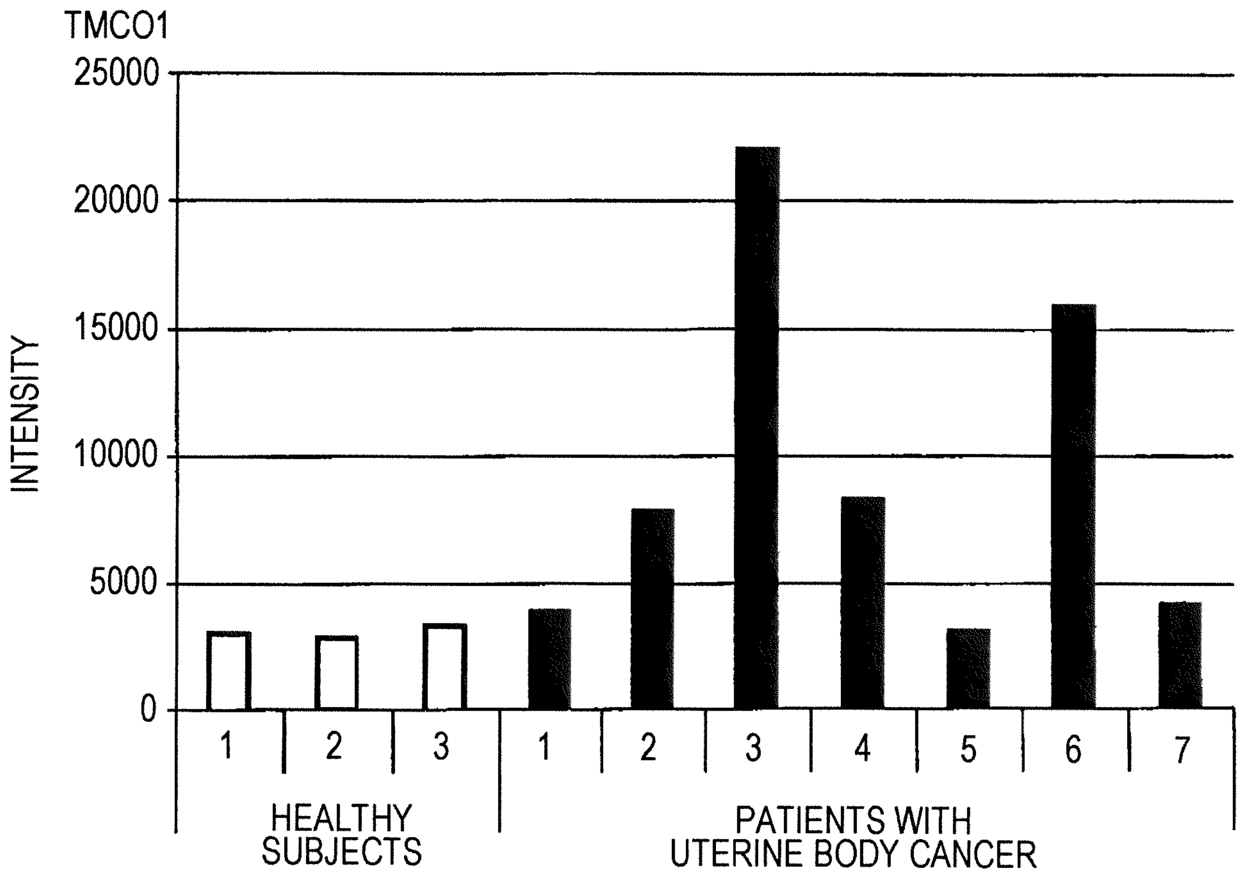
TMCO1
INTENSITY
25000
20000
15000
10000
5000
0
1
2
3
1
2
3
4
5
6
7
HEALTHY SUBJECTS
PATIENTS WITH UTERINE BODY CANCER

FIG. 5B

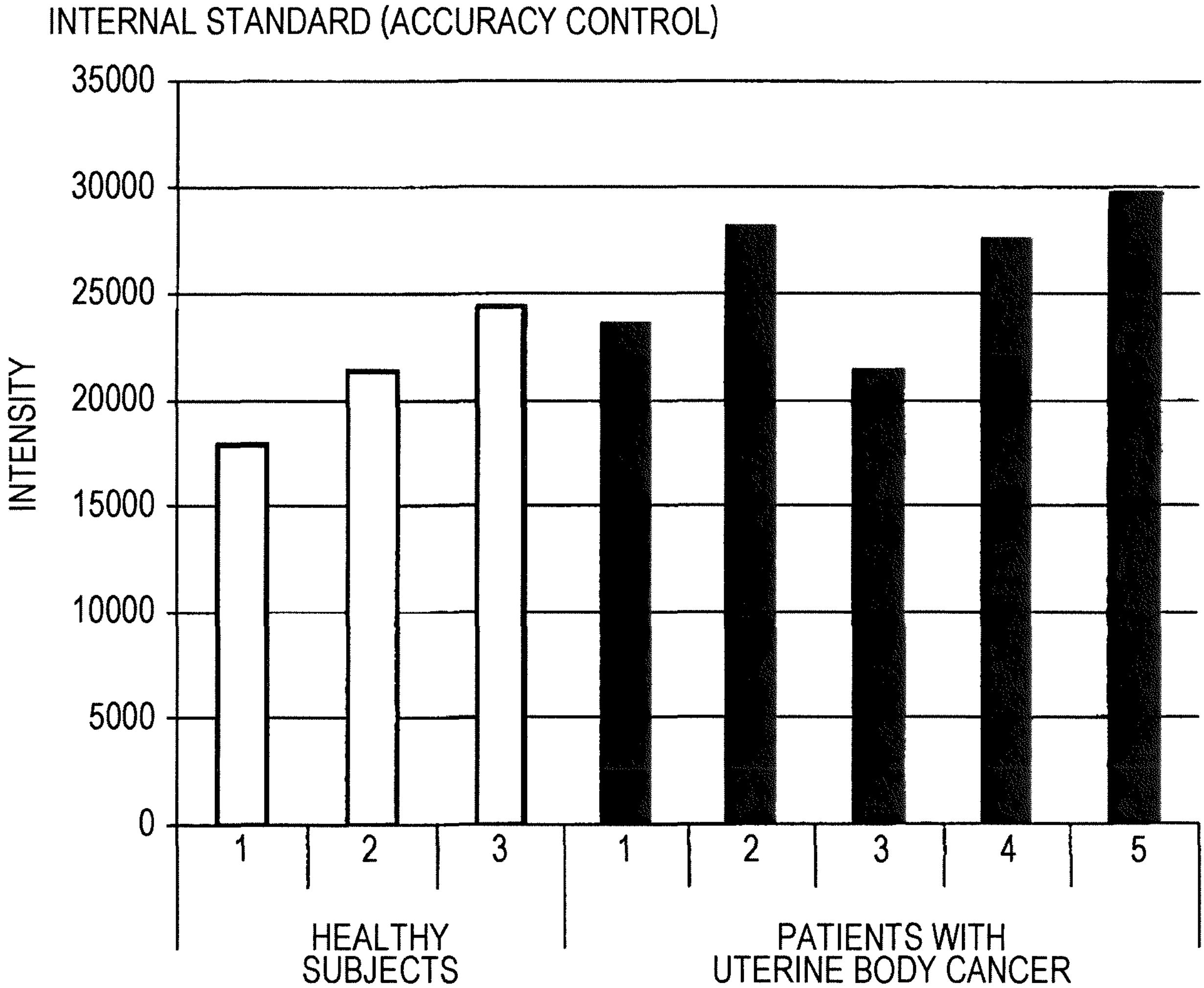
INTERNAL STANDARD (ACCURACY CONTROL)
INTENSITY
35000
30000
25000
20000
15000
10000
5000
0
1
2
3
1
2
3
4
5
HEALTHY SUBJECTS
PATIENTS WITH UTERINE BODY CANCER

FIG. 6

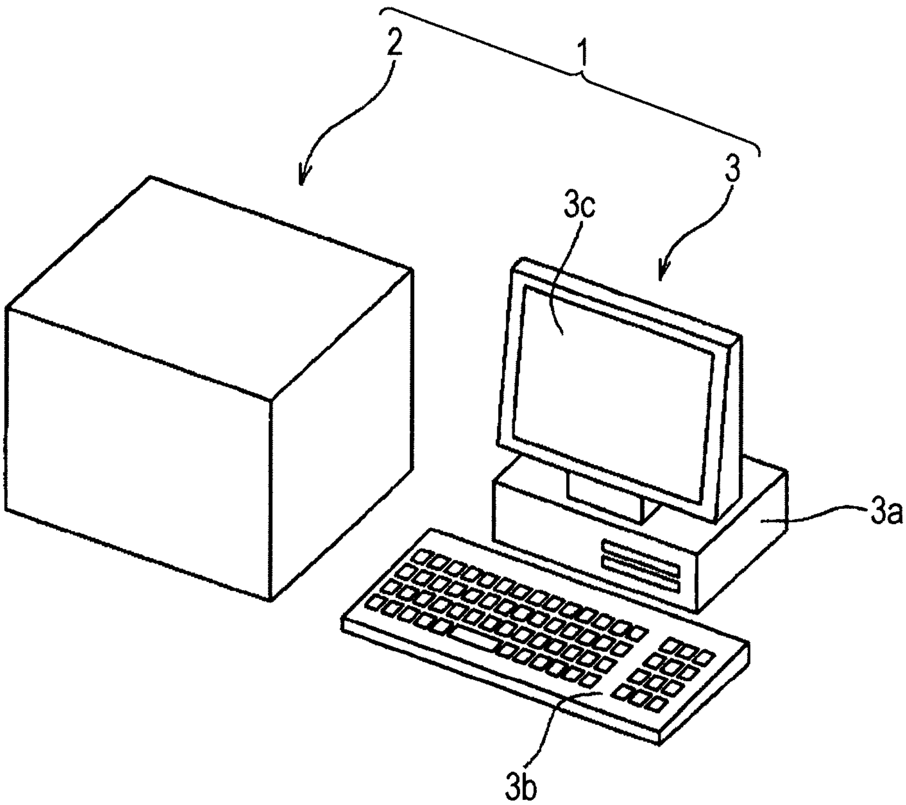

FIG. 7

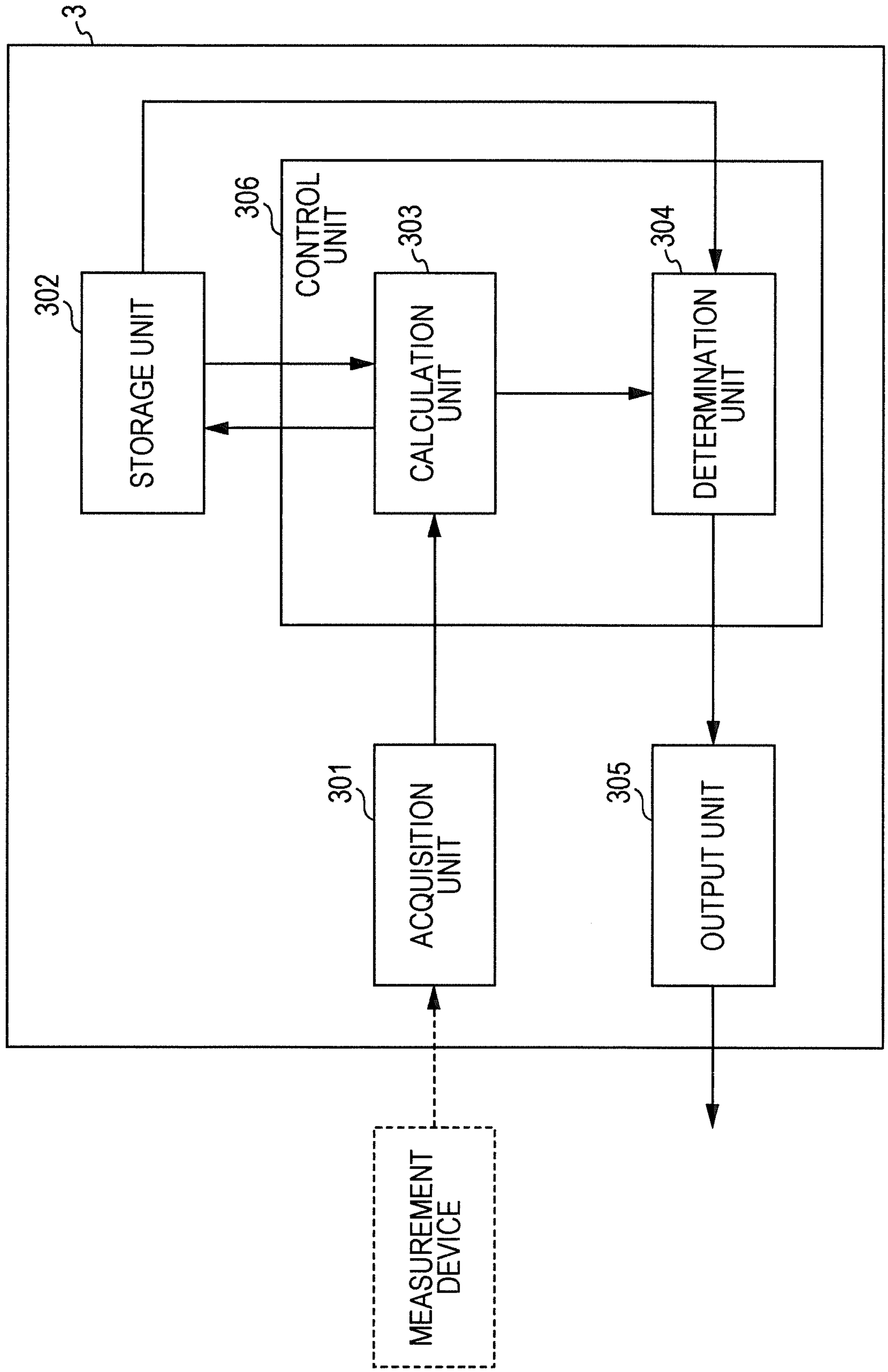
3
302
STORAGE UNIT
306
CONTROL UNIT
303
CALCULATION UNIT
304
DETERMINATION UNIT
301
ACQUISITION UNIT
305
OUTPUT UNIT
MEASUREMENT DEVICE

FIG. 8

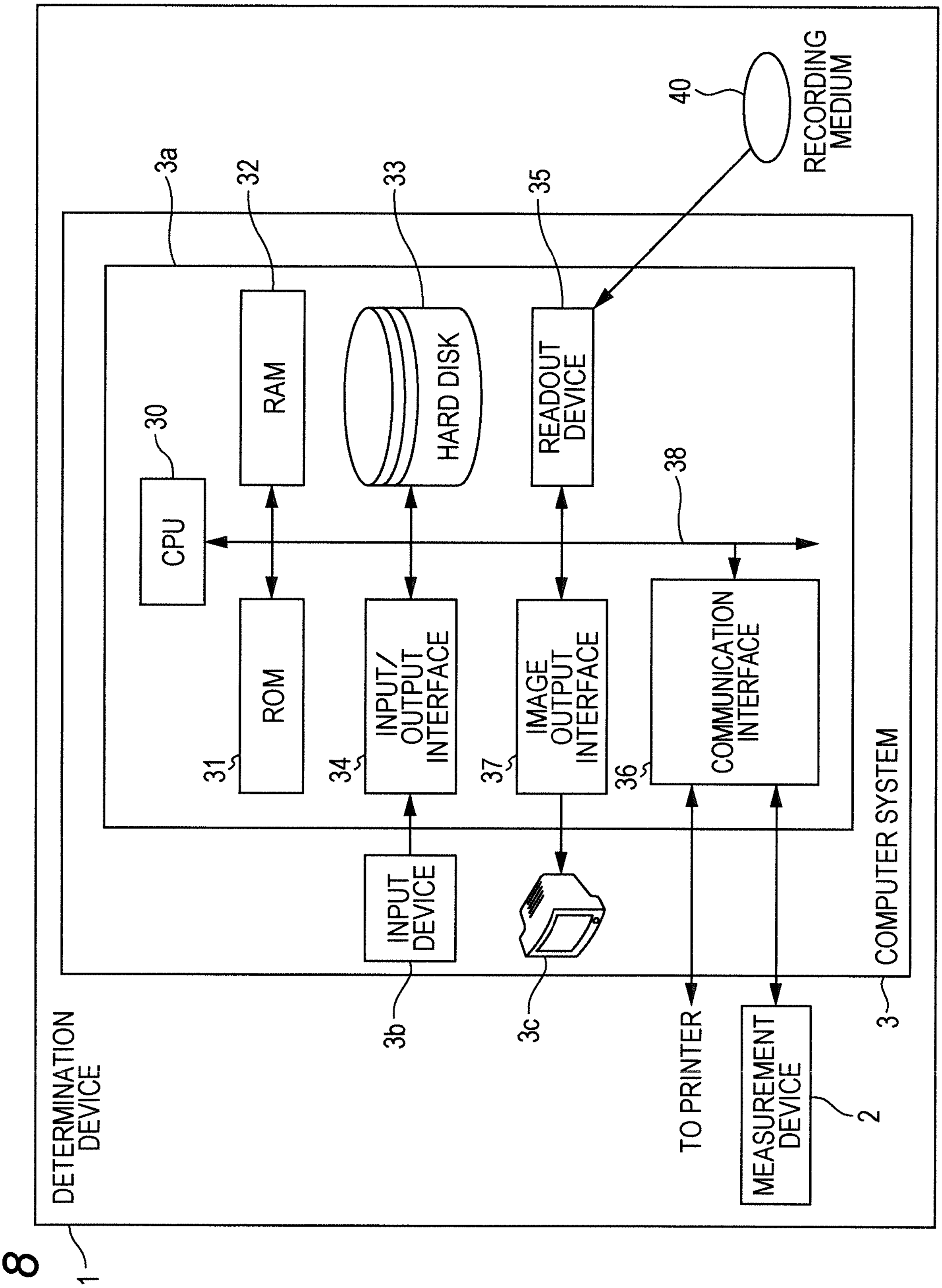
DETERMINATION DEVICE
1
CPU
30
RAM
32
ROM
31
HARD DISK
33
INPUT/ OUTPUT INTERFACE
34
READOUT DEVICE
35
IMAGE OUTPUT INTERFACE
37
COMMUNICATION INTERFACE
36
38
3a
RECORDING MEDIUM
40
INPUT DEVICE
3b
3c
COMPUTER SYSTEM
3
TO PRINTER
MEASUREMENT DEVICE
2

FIG. 9

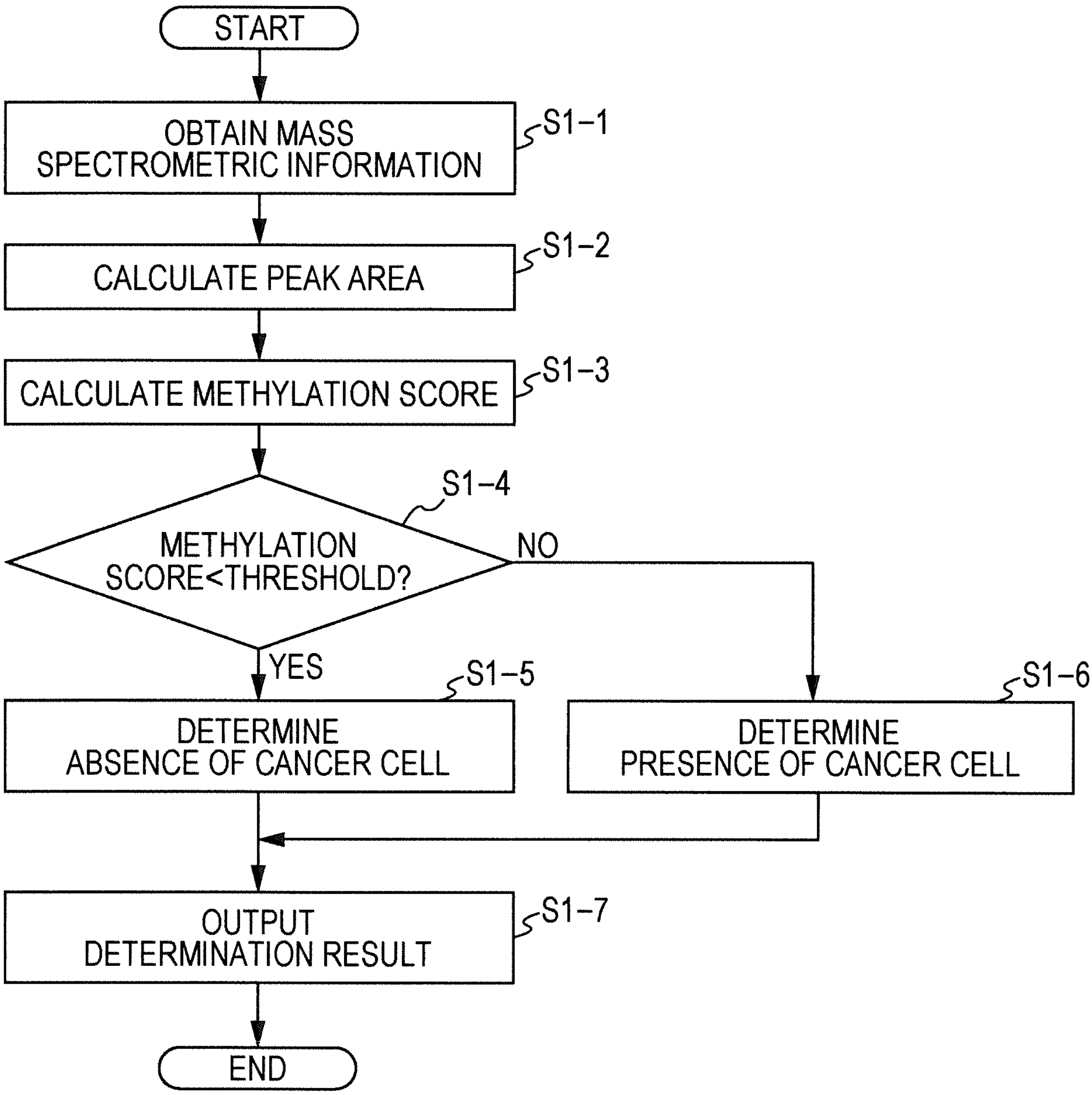
START
OBTAIN MASS SPECTROMETRIC INFORMATION
S1-1
CALCULATE PEAK AREA
S1-2
CALCULATE METHYLATION SCORE
S1-3
METHYLATION SCORE<THRESHOLD?
S1-4
NO
YES
DETERMINE ABSENCE OF CANCER CELL
S1-5
DETERMINE PRESENCE OF CANCER CELL
S1-6
OUTPUT DETERMINATION RESULT
S1-7
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014142497 A


### Non-patent literature cited in the description


- **ESTELLER M. et al.** *AM. J. Pathol.,* 1999, vol. 155, 1767-1772
- **FURLAN D.** *Clin. Cancer Res.,* 2006, vol. 12, 3329-3336
- **SELAMAT SA ; CHUNG BS ; GIRARD L ; ZHANG W et al.** Genome-scale analyses of DNA methylation in lung adenocancer and integration with mRNA expression. *Genome Res.,* July 2012, vol. 22 (7), 1197-211
- **KOBAYASHI Y ; ABSHER DM ; GULZAR ZG ; YOUNG SR ; MCKENNEY JK ; PEEHL DM ; BROOKS JD ; MYERS RM ; SHERLOCK G.** DNA methylation profiling reveals novel biomarkers and important roles for DNA methyltransferases in prostate cancer. *Genome Res.,* July 2011, vol. 21 (7), 1017-27
- **SALHIA B ; BAKER A ; AHMANN G ; AUCLAIR D ; FONSECA R ; CARPTEN J.** DNA methylation analyses determines the high frequency of genic hypomethylation and low frequency of hypermethylation events in plasma cell tumors. *Cancer Res.,* 01 September 2010, vol. 70 (17), 6934-44